# EUROPEAN PATENT SPECIFICATION

(11) **EP 1 765 351 B1**
(45) Date of publication and mention of the grant of the patent: **04.03.2009**
(21) Application number: 05744735.1
(22) Date of filing: 09.06.2005
(51) Int. Cl.: A61K 31/519, A61K 31/695, A61P 35/00

(54) **PROTEIN KINASE INHIBITORS**
PROTEINKINASEINHIBITOREN
INHIBITEURS DE PROTEINE KINASE

(30) Priority: 09.06.2004 EP 04013572
(43) Date of publication of application: 28.03.2007
(73) Proprietor: Oncalis AG, 8952 Schlieren (CH)
(72) Inventor: BERSET, Catherine, CH-5210 Windisch (CH); AUDETAT, Stephan, CH-8107 Buchs (CH); TIETZ, Julia, CH-8952 Schlieren (CH); GUNDE, Tea, CH-8005 Zürich (CH); BARBERIS, Alcide, CH-8057 Zürich (CH); SCHUMACHER, Andreas, 79588 Efringen-Kirchen (DE); TRAXLER, Peter, CH-4124 Schönenbuch (CH)
(74) Representative: Blum, Rudolf Emil
(86) International application number: PCT/CH2005/000321
(87) International publication number: WO 2005/120513

(56) References cited:
- WO-A-03/018589
- US-A1- 2003 018 032
- US-A1- 2003 199 525

## Description

The present invention relates to protein kinase inhibitors, in particular receptor tyrosine kinase inhibitors, and their use for the treatment of diseases involving a protein kinase such as hyperproliferative disorders e.g. cancer.

### Background Art

US2003 / 0199525 discloses certain pyrazolo-pyrimidines and their use as kinase inhibitors; US 2003 / 0018032 discloses certain imidazo-3yl-amines and their use as analgetics.

Protein kinases, in particular receptor protein tyrosine kinases (RTK), are key regulators of intercellular communication that controls cell growth, proliferation, differentiation, survival and metabolism. About 20 different RTK families have been identified that share a similar structure, namely an extracellular binding site for ligands, a transmembrane region and an intracellular tyrosine kinase domain. Extracellular ligand binding induces or stabilizes receptor dimerization leading to increased RTK kinase activity. The intracellular catalytic domain displays the highest level of conservation among RTKs and includes the ATP-binding site that catalyzes protein phosphorylation of e.g. cytoplasmic tyrosine residues, which serve as docking sites for Src homology 2 (SH2)-and phosphotyrosine-binding (PTB) domain-containing proteins such as Grb2, Shc, Src, Cbl or phospholipase C γ. These proteins subsequently recruit additional effectors containing SH2, SH3, PTB and pleckstrin-homology (PH) domains to the activated receptor, which results in the assembly of signaling complexes at the membrane and the activation of a cascade of intracellular biochemical signals. The most important downstream signaling cascades activated by RTKs include the Ras-extracellular regulated kinase (ERK)-mitogen activated (MAP) kinase pathway, the phosphoinositide 3-kinase (PI 3-kinase)-Akt and the JAK/STAT pathway. The complex signaling network triggered by RTKs eventually leads either to activation or repression of various subsets of genes and thus defines the biological response to a given signal.

The activity of RTKs and their mediated cellular signaling is precisely coordinated and tightly controlled in normal cells. Deregulation of the RTK signaling system, either by stimulation through growth factor and/or through genetic alteration, result in deregulated tyrosine kinase activity. These aberrations generally result in RTKs with constitutive or strongly enhanced kinase activity and subsequent signaling capacity, which leads to malignant transformation. Therefore, they are frequently linked to human cancer and also to other hyperproliferative diseases such as psoriasis. The most important mechanisms leading to constitutive RTK signaling include overexpression and/or gene amplification of RTKs, genetic alterations such as deletions and mutations within the extracellular domain as well as alterations of the catalytic site, or autocrine-paracrine stimulation through aberrant growth factor loops.

For example, in many human cancers, gene amplification and/or overexpression of RTKs occurs, which might increase the response of cancer cells to normal growth factor levels. Additionally, overexpression of a specific RTK on the cell surface increases the incidence of receptor dimerization even in the absence of an activating ligand. In many cases this results in constitutive activation of the RTK leading to aberrant and uncontrolled cell proliferation and tumor formation. An important example for such a scenario is HER2, also known as ErbB2, that belongs to the epidermal growth factor (EGF) receptor family of RTKs. Overexpression of HER2 was found in various types of human cancers, especially in human breast and ovarian carcinomas. Most importantly, aberrantly elevated levels of HER2 correlate with more aggressive progression of disease and reduced patient survival time. EGFR, which was the first receptor tyrosine kinase to be molecularly cloned, also plays a fundamental role in tumorigenesis. EGFR is frequently overexpressed in non-small-cell lung, bladder, cervical, ovarian, kidney and pancreatic cancer and in squamous-cell carcinomas of the head and neck. The predominant mechanism leading to EGFR overexpression is gene amplification with up to 60 copies per cell reported in certain tumors. In general, elevated levels of EGFR expression are associated with high metastatic rate and increased tumor proliferation.

Since protein kinases, in particular tyrosine kinases, have been implicated in a variety of cancer indications, RTKs and the activated signaling cascades represent promising areas for the development of target-selective anticancer drugs. One approach to inhibit aberrant RTK signaling is the development of small-molecule drugs that selectively interfere with their intrinsic tyrosine kinase activity and thereby block receptor autophosphorylation and activation of downstream signal transducers.

### Disclosure of the invention

Hence, it is a general object of the present invention to provide compounds having a protein kinase inhibitory activity which can be used for the treatment of disorders involving a protein kinase such as hyperproliferative diseases.

Now, in order to implant this and still further objects of the invention, which become more readily apparent as the description proceeds, said protein kinease inhibitory activity is provided by a compound being used for the treatment of disorders involving a protein kinase, in particular a tyrosine kinase, of formula I, 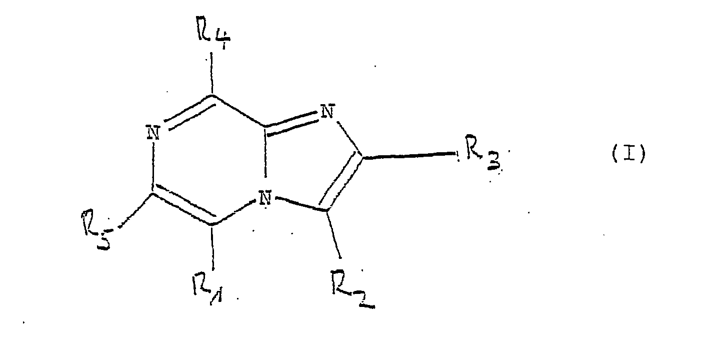 wherein R₁, R₂ and R₄ are independently selected from hydrogen, optionally substituted C₁-C₆ alkyl, optionally substituted C₃-C₈ cycloalkyl, NR₆R₇, OR₆, SR₆, (CH)ₘR₆R₇, wherein R₆ and R₇ are independently selected from hydrogen, optionally substituted C₃-C₈ cycloalkyl, optionally substituted aryl, optionally substituted 5- or 6- membered heterocycle, (CH₂)ₙCO(O)R₈, (CH₂)ₘR' where n = 0, 1, 2, 3, 4 and wherein R₈ is hydrogen, C₁-C₆ alkyl, C₁-C₆ alkoxy, optionally substituted C₃-C₈ cycloalkyl, optionally substituted aryl and wherein R' is selected from hydrogen, C₁-C₆ alkyl, C₁-C₆ alkoxy, optionally substituted C₃-C₈ cycloalkyl, optionally substituted aryl, halogen, hydroxyl, NO₂, NH₂, SO₂NH₂, cyano and m is 0, 1, 2, 3, 4;
R₃ is hydroxyl, halogen, NH₂, NO₂, cyano, SH, optionally substituted C₁-C₆ alkyl, optionally substituted C₃-C₈ cycloalkyl, optionally substituted aryl, optionally substituted 5- or 6-membered heterocycle,
R₅ is selected from C₁-C₆ alkyl, hydrogen, hydroxyl, alkoxy, NH₂, halogen, cyano, alkine, COOR" wherein R" is selected from hydrogen, C₁-C₆ alkyl, C₃-C₈ cycloalkyl,
Preferred compounds of formula I are those where R₁ is hydrogen, methyl, ethyl, propyl, cyclopropyl, NH₂, SH and R₂ is NR₆R₇ wherein R₆ is hydrogen, R₇ is selected from C₃-C₈ cycloalkyl, optionally substituted phenyl, (CH₂)ₙCO(O)R₈ where n = 1, and wherein R₈ is C₁-C₆ alkyl, C₁-C₆ alkoxy, R₃ is optionally substituted phenyl, optionally substituted 5- or 6-membered heterocyle, cyano;
R₄ is hydrogen, NH(CH₂)ₘR', O(CH₂)ₘR', S(CH₂)ₘR' wherein R' is selected from optionally substituted phenyl, optionally substituted C₃-C₈ cycloalkyl and m = 1, 2,
R₅ is methyl, hydrogen, hydroxyl.

Preferred compounds of formula I are those where R₁ is hydrogen,
R₂ is NHR₇ wherein R₇ is selected from C₃-C_{B} cycloalkyl, phenyl optionally substituted with a substituent selected from C₁-C₆ alkyl, C₁-C₆ alkoxy, halogen, hydroxyl, NH₂, SO₂NH₂; (CH₂)ₙCO(O)R₈ where n = 1, and wherein R₈ is methyl, ethyl, propyl, butyl,
R₃ is phenyl optionally substituted with a substituent selected from C₁-C₆ alkyl, C₁-C₆ alkoxy, halogen, hydroxyl, NO₂ optionally substituted 5- or 6-membered heterocycle, cyano, R₄ and R₅ are hydrogen, or those where
R₁ is hydrogen,
R₂ is NR₆R₇ wherein R₆ is hydrogen, R₇ is optionally substituted phenyl,
R₃ is optionally substituted phenyl or optionally substituted, preferably unsubstituted, furyl,
R₄ is hydrogen, methyl or NR₆R₇ wherein R₆ and R₇ are independently from each other, selected from hydrogen, C₁-C₂ alkyl, optionally substituted by phenyl, and
R₅ is hydrogen.

Even more preferred compounds of formula I are those where
R₁ is hydrogen,
R₂ is NHR₇ wherein R₇ is selected from phenyl
optionally substituted with a radical selected from methyl, ethyl, propyl, halogen, methoxy, hydroxyl,
R₃ is phenyl optionally substituted with a substituent selected from methoxy, bromo, chloro, fluoro, hydroxyl, NO₂, NH₂, R₄ and R₅ are hydrogen, or those where
R₁ is hydrogen,
R₂ is -NR₆R₇ wherein R₆ is hydrogen and R₇ is unsubstituted phenyl or phenyl substituted in 3 and/or 4 position by one or two substituents independently selected from halogen, C₁-C₂ alkoxy, in particular methoxy, C₁-C₂ alkyl, in particular methyl, CF₃, NO₂, NH₂, NHCH₃, N(CH₃)₂,
R₃ is unsubstituted phenyl or phenyl substituted in 3 and/or 4 position by a substituent independently selected from C₁-C₄ alkoxy, or hydroxy, or amino, or the substituents in 3- and 4-position form together a 5- or 6-membered heterocycle,
R₄ is hydrogen or methyl and
R₅ is hydrogen.

Especially preferred compounds of formula I are those where R₁ is hydrogen, R₂ is NHR₇ wherein R₇ is phenyl, R₃ is phenyl and R₄ and R₅ are hydrogen; and where
R₁ is hydrogen, R₂ is NHR₇ wherein R₇ is phenyl, R₃ is phenyl substituted with hydroxyl and R₄ and R₅ are hydrogen, or those where
R₁ is hydrogen,
R₂ is -NR₆R₇ wherein R₆ is hydrogen and R₇ is
unsubstituted phenyl or phenyl substituted in 3 and/or 4 position by one or two substituents independently selected from halogen and CF₃, in particular an unsubstituted phenyl or a phenyl substituted in 3 or 4 position,
R₃ is unsubstituted phenyl or phenyl substituted in 3 and/or 4 position by methoxy or hydroxy, in particular an unsubstituted phenyl or a phenyl substituted in 3- or 4-position,
R₄ is hydrogen or methyl, and
R₅ is hydrogen.

A presently especially preferred compound is a compound of formula I (see also Example 1) where
R₁ is hydrogen,
R₂ is -NR₆R₇ wherein R₆ is hydrogen and R₇ is phenyl substituted with halogen in 3-position,
R₃ is unsubstituted phenyl,
R₄ is methyl, and
R₅ is hydrogen.

The compounds of the present invention are preferably used for the treatment of a disease which involves a tyrosine kinase, preferably a receptor tyrosine kinase.

The compounds of the present invention are particularly suitable for the treatment of a disease involving a member of the eph family of receptor tyrosine kinases. A preferred member of the eph family is ephrin B4.

The compounds of the present invention can be used for the treatment of many diseases involving a protein kinase such as hyperproliferative diseases, in particular cancer.

In a second aspect the present invention relates to the use of a compound of formula Ia: 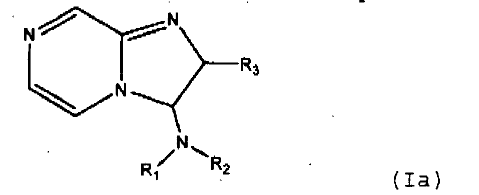 wherein R₁ is C₃-C₈ cycloalkyl, phenyl optionally substituted with a substituent selected from C₁-C₆ alkyl, SO₂NH₂ halogen, C₁-C₆ alkoxy; (CH₂)ₙCO(O)R₄ where n = 1, 2, 3, 4 and wherein R₄ is hydrogen, optionally substituted C₁-C₆ alkyl, optionally substituted C₃-C₈ cycloalkyl, optionally substituted aryl,
R₂ is hydrogen, C₁-C₆ alkyl and
R₃ is phenyl optionally substituted with a substituent selected from C₁-C₆ alkyl, C₁-C₆ alkoxy, halogen, hydroxyl, NO₂
for the manufacture of a medicament for the treatment of a hyperproliferative disease or a malignant transformation involving a protein kinase.

Preferred compounds of formula la are those where
R₁ is cyclopentyl, cyclohexyl, phenyl optionally substituted with a substituent selected from methyl, ethyl, propyl; (CH₂)ₙCO(O)R₄ where n = 1 and wherein R₄ is methyl, ethyl, propyl,
R₂ is hydrogen and
R₃ is phenyl optionally substituted with a substituent selected from methoxy, ethoxy, hydroxyl, N0₂, bromine, fluorine, chlorine.

Even more preferred compounds of formula Ia are those where
R₁ is phenyl, R₂ is hydrogen and R₃ is phenyl substituted with hydroxyl and
R₁ is phenyl, R₂ is hydrogen and R₃ is phenyl.

In a third aspect, the present invention provides a compound of formula Ib as medicament 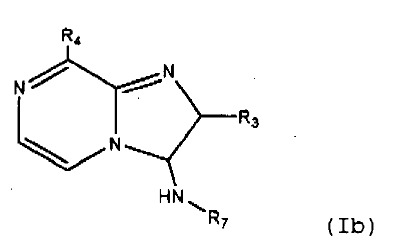 wherein R₇ is optionally substituted phenyl, R₃ is optionally substituted phenyl or optionally substituted, preferably unsubstituted, furyl, and R₄ is methyl or NR6R7 wherein R6 and R₇ are independently from each other selected from hydrogen, C₁-C₂ alkyl, optionally substituted by phenyl.

Preferred compounds of formula Ib are those where R₇ is unsubstituted phenyl or phenyl substituted in 3 and/or 4 position by one or two substituents independently selected from halogen, C₁-C₂ alkoxy, in particular methoxy, C₁-C₂ alkyl, in particular methyl, CF₃, NO₂, NH₂, NHCH₃, N(CH₃)₂; R₃ is unsubstituted phenyl or phenyl substituted in 3 and/or 4 position by a substituent independently selected from C₁-C₄ alkoxy, or hydroxy, or amino, or the substituents in 3- and 4-position form together a 5- or 6-membered heterocycle, and R₄ is methyl.

Even more preferred compounds are those compounds of formula Ib where R₇ is unsubstituted phenyl or phenyl substituted in 3 and/or 4 position by one or two substituents independently selected from halogen, and CF₃, in particular an unsubstituted phenyl or a phenyl substituted in 3 or 4 position; R₃ is unsubstituted phenyl or phenyl substituted in 3 and/or 4 position by methoxy or hydroxy, in particular an unsubstituted phenyl or a phenyl substituted in 3- or 4-position, and R₄ is methyl.

Even more preferred compounds are those compounds of formula Ib where R₇ is phenyl substituted with halogen in 3-position; R₃ is unsubstituted phenyl, and R₄ is methyl.

Preferably said disorder is a hyperproliferative disorder, in particular a cancer.

The compounds of the present invention can as well be used as research tools in functional genomics, drug discovery, target validation and *ex vivo* diagnostics.

### Modes for carrying out the invention

In the context of the present invention it has been surprisingly found that the compounds of formula I have besides their known analgetic activity as well a protein kinase modulating activity, in particular a tyrosine kinase inhibiting activity.

The compounds of the present invention are preferably used for the treatment of a hyperproliferative disease, in particular cancer. The compounds of the present invention are particularly suitable for the treatment of a hyperproliferative disorder involving a receptor tyrosine kinase of the eph family, preferably eph B4, or a tumor involving the cytoplasmic tyrosine kinase src. The src non-receptor tyrosine kinase in known to be involved in the development of various cancers. For a review see the publication of Warmuth et al., Curr. Pharm. Des. 2003: 9(25):2043-59.

There exists evidence that receptor tyrosine kinases of the eph family are involved in the development of tumors such as breast cancer, liver cancer, gastrointestinal cancer, neuroblastomas, leukemias and lymphomas, prostate cancer, pancreatic cancer, lung cancer, melanoma, ovarian cancer, thyroid cancers, sarcomas, renal carcinomas and epidermoid cancer (M. Nakamoto et al, Microscopy Research and technique 59:58-62 (2002)). Therefore, the compounds of the present invention can preferably be used for the treatment of the above mentioned types of tumors.

The term "protein kinase" as used herein encompasses all types of protein kinases such as serine/threonine kinases, tyrosine kinases, receptor tyrosine kinases and non-receptor tyrosine kinases.

The compounds of the present invention having formula I can be prepared by methods described e.g. in WO 01/27111 or in WO 03/031447. The compounds of the present invention having formula II can be prepared by methods described e.g. in US 2003/0225098.

The compounds of the invention can be administered in a variety of dosage forms, e.g. orally, in the form of tablets, capsules, sugar- or film-coated tablets, liquid solutions or suspensions; rectally, in the form of suppositories; parenterally, e.g. intramuscularly, or by intravenous injection of infusion; or topically. The dosage depends on the age, weight, condition of the patient and administration route.

The pharmaceutical compositions containing the compounds of the invention are usually prepared following conventional methods and are administered in a pharmaceutically suitable form.

For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate, effervescing mixtures; dyestuffs; sweeteners; wetting agents, such as lecithin, polysorbates, laurylsulphates; and, in general, non-toxic and pharmacologically inactive substances used in pharmaceutical formulations. Said pharmaceutical preparations may be manufactured in known manner, for example by means of mixing, granulating, tabletting, sugar-coating or filmcoating processes.

The liquid dispersion for oral administration may be, e.g., syrups, emulsions and suspensions.

The syrup may contain as carrier, for example, saccharose or saccharose with glycerin and/or mannitol and/or sorbitol.

The suspensions and the emulsions may contain as carrier, for example, a natural gum, agar, sodium alginate, pectin, methylcellulose, carboxymethylcellulose or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride.

The solutions for intravenous injections or infusion may contain as carrier, for example, sterile water or, preferably, they may be in the form of sterile aqueous, isotonic saline solutions.

The suppositories may contain, together with the active compound, a pharmaceutically acceptable carrier, e.g. cocoa-butter, polyethylene glycol, a polyoxyethylene sorbitan fatty acid ester surfactant or lecithin.

Compositions for topical application, e.g. creams, lotions or pastes, can be prepared by admixing the active ingredient with a conventional oleaginous or emulsifying excipient.

The compounds of the present invention may be administered to a patient in form of phamaceutically acceptable salts. Suitable pharmaceutically-acceptable salts include acid addition salts such as methanesulfonate, fumarate, hydrochloride, citrate, maleate, tartrate and hydrobromide. Also suitable are salts formed with phosphoric and sulfuric acid. Further suitable salts are base salts such as an alkali metal salt for example sodium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine, tris-(2-hydroxyethyl) amine, N-methyl d-glucamine and amino acids such as lysine. There may be more than one cation or anion depending on the number of charged functions and the valency of the cations or anions.

The compounds of the present invention may be administered in the form of a pro-drug which is broken down in the human or animal body to give a compound of the present invention. A prodrug may be used to alter or improve the physical and/or pharmacokinetic profile of the parent compound and can be formed when the parent compound contains a suitable group or substituent which can be derivatised to form a prodrug. Examples of prodrugs include in-vivo hydrolysable esters of a compound of the present invention or a pharmaceutically-acceptable salt thereof.

Below are shown three preferred compounds of the present invention:

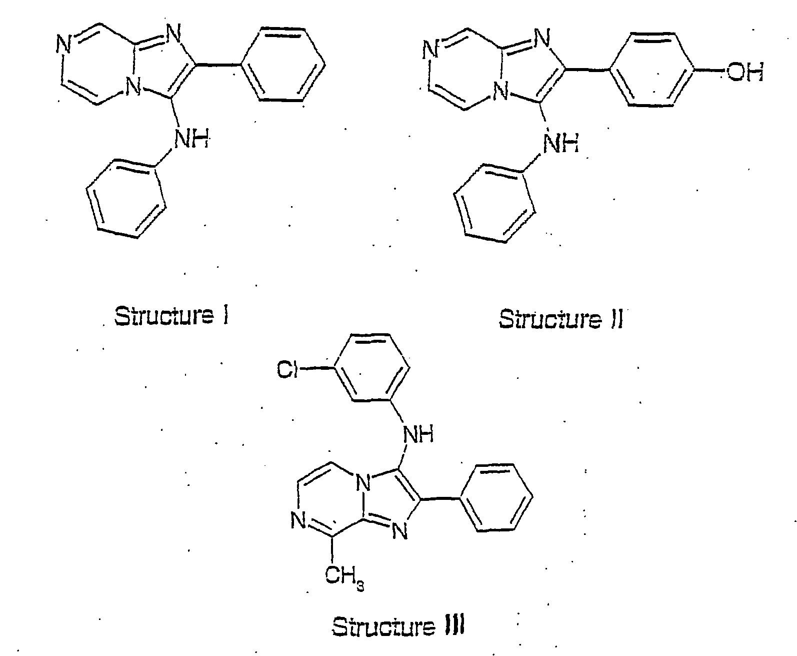

### EXAMPLES

The invention is further illustrated by the following preparations and examples wich should not be construed to limit the scope of the disclosure. Alternative pathways and analogous structures will be apparent to those skilled in the art.

The following abbreviations are used in the following examples:
rt = room temperature (ca. 25°C)
Rt = Retention time
AcOH = acetic acid
EtOAc = ethyl acetate
EtOH = ethanol
MeOH = methanol
TFA = trifluoro acetic acid
NMR = nuclear magnetic resonance spectroscopy
HPLC = high pressure liquid chromatography
LC/MS = liquid chromatography mass spectrometry
RP = reverse phase

The following purification methods were applied to obtain pure samples: Crystallization from typical organic solvents, flash chromatography on silica gel, preparative HPLC on RP-silica gel and any combinations thereof.

For preparative HPLC, an Agilent Series 1100 Instrument with a Zorbax SB-C18 column , 21.2 x 250 mm, 7 µ, was used; solvents CH₃CN - water (0.1 % TFA each).

Where HPLC data are presented, analysis was done on a Agilent Series 1100 Instrument with a Supelco Discovery C18 column (4.6 x 50 mm, 5 µ, detecting at 254 nm and 220 nm; gradient 10 % to 99 % CH₃CN within 4 minutes, 1 min. at 99 % CH₃CN using CH₃CN - water (0.1 % TFA each) solvent system with a flow rate of 2.0 mL / min. The retention times in minutes are given.

Where NMR Data are presented, ¹H spectra were obtained on a Bruker DPX 300 (300.13 MHz) and are reported as ppm down field from tetramethylsilane with the number of protons.

Where LC/Ms data are presented, analysis was performed using a Micromass ZQ, (150-1000 u), ESI-positive spectrometer and Agilent Series 1100 Instrument, with a YMC-Pack ProC 18 (3 µm), 33x3 mm column; gradient flow 5 % CH₃CN /methanol/ 95% water/ 0.05% formic acid to 100% CH₃CN /methanol/ 0% water/ 0.05% formic acid within 3 minutes using CH₃CN /methanol (80:20) - water (0.05% HCOOH) as solvent system with a flow rate of 1.7 mL/min. The retention times in minutes and observed parent ion are given.

### INTERMEDIATE 1. 3-Methyl-pyrazin-2-ylamine

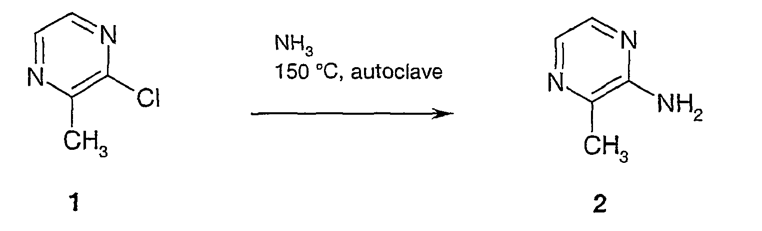

In an autoclave, 2-chloro-3-methyl-pyrazine (1) (10.0 g, 77.8 mmol) was dissolved in dry methanol (30 mL). Ammonia gas (60 g) was added. The mixture was heated to 150°C for 8 hours. (start pressure: 10 bar, end pressure: 90 bar). After cooling to rt, the mixture was evaporated to a brown solid, which was dissolved in 1N hydrochloric acid (100 mL) and washed with dichloromethane. The aqueous layer was slowly poured on cold saturated aqueous ammonia (150 mL), then extracted with dichloromethane (3 x 100 mL). The combined organic layers were dried (Na₂SO₄) and evaporated. The product was extracted from the residual solid with hot acetone (200 mL). Evaporation yielded 36 % of 3-methyl-pyrazin-2-ylamine **(2)** as a yellow solid.

### EXAMPLE 1: (3-Chloro-phenyl)-(8-methyl-2-phenyl-imidazo[l,2-a]pyrazin-3-yl)-amine (3)

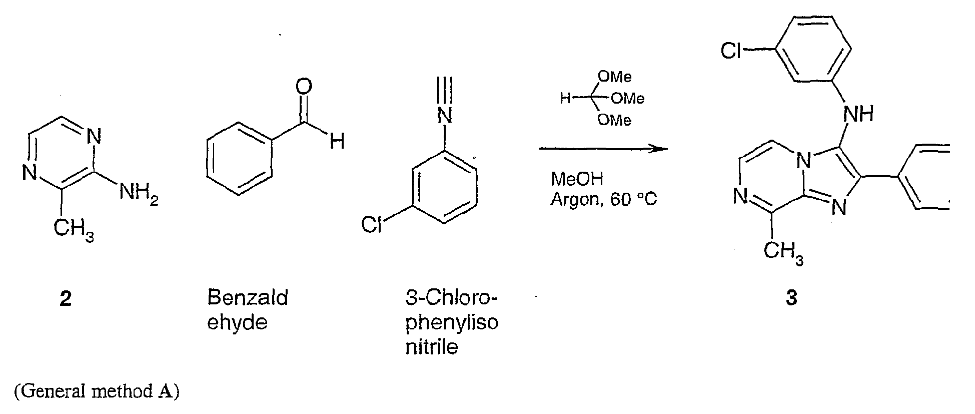

3-Methyl-pyrazin-2-ylamine **(2)** (109 mg, 1.0 mmol), benzaldehyde (106 mg, 1.0 mmol) and 3-chlorophenylisonitrile (138 mg, 1.0 mmol) were dissolved in a mixture of dry methanol (2.0 mL) and trimethyl orthoformate (2.0 mL) under argon. The mixture was stirred at 60°C for 3 hours, then cooled to rt. An analytically pure sample of 3 was obtained from the crude product using preparative HPLC.

### EXAMPLE 2: (3,4-Dichloro-phenyl)-(2-phenylimidazo[1,2-a]pyrazin-3-yl)-amine (29)

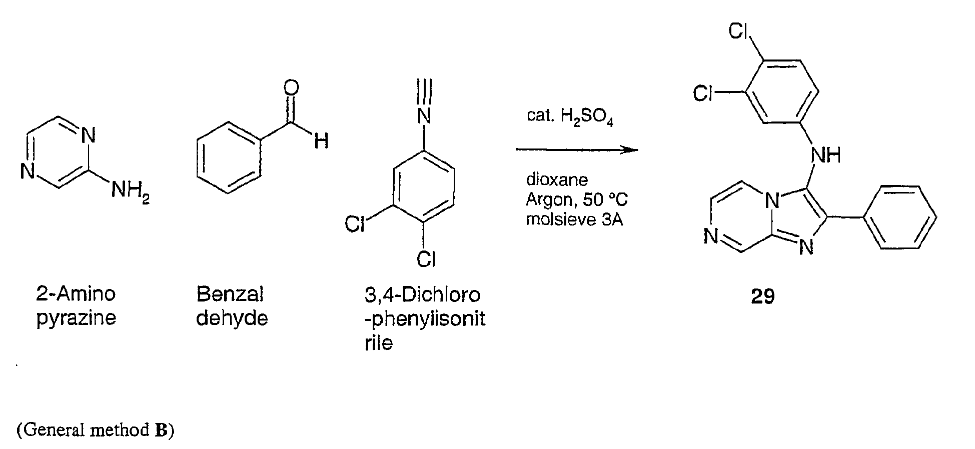

2-Amino-pyrazine (95 mg, 1.0 mmol) and benzaldehyde (106 mg, 1.0 mmol) were dissolved in dry dioxane (2.0 mL) containing molecular sieves (3Å) under argon. After 5 minutes sulfuric acid (20µL) and 3,4-dichloro-phenylisonitrile (172 mg, 1.0 mmol) were added. The mixture was then stirred at 50°C for 3 hours, cooled to rt and filtered. The product **29** was obtained by crystallization from acetonitrile.

### EXAMPLE 3: N'8-Benzyl-N'3-(3-chloro-phenyl)-2-phenyl-imidazo[1,2-a]pyrazine-3,8-diamine (22)

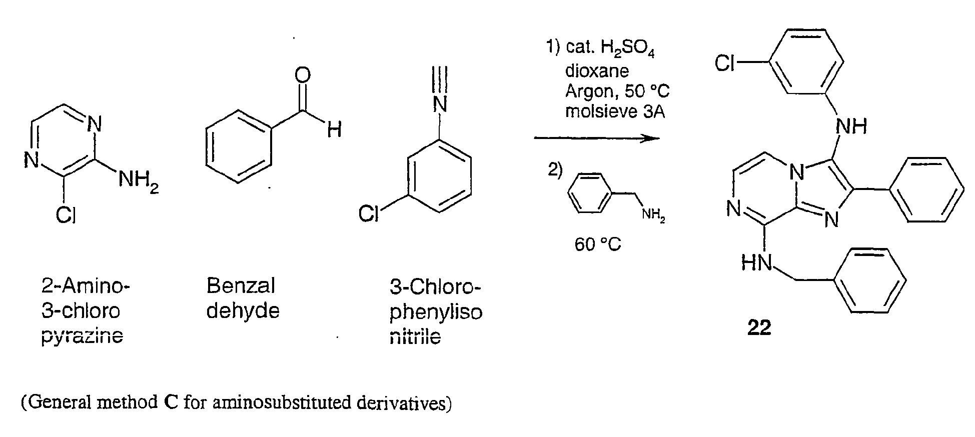

2-Amino-3-chloro-pyrazine (130 mg, 1.0 mmol) and benzaldehyde (106 mg, 1.0 mmol) were dissolved in dry dioxane (3.0 mL) containing molecular sieves (3Å) under Argon. After 5 minutes sulfuric acid (20µL) and 3-chlorophenylisonitrile (138 mg, 1.0 mmol) were added. The mixture was then stirred at 50°C for 3 hours, cooled to rt and filtered.

Benzylamine (536 mg, 5.0 mmol) was added to the filtrate and the mixture was heated to 60°C for 20 h. After cooling to rt, filtration and evaporation to dryness, an analytically pure sample of **22** was obtained from the crude product using preparative HPLC.

### EXAMPLE 4: [2-(4-Amino-phenyl)-imidazol[1,2-a]pyrazin-3-yl]-(3-chloro-phenyl)-amine (10)

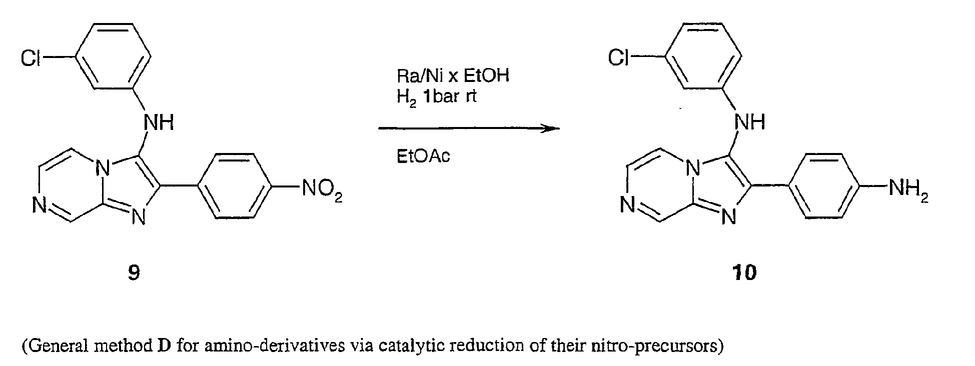

**9** (82 mg, 0.22 mmol) was dissolved in ethyl acetate (20 mL). 50 mg of Ra/Ni x EtOH were added and the mixture was hydrogenated (1 bar) at rt for 40 hours. The catalyst was filtered off, washed with ethyl acetate and the filtrate was evaporated to yield product **10** as a yellow solid. Pure **10** was obtained by crystallization from acetonitrile.

**9** was prepared in analogy to General Method **A** using 4-nitrobenzaldehyde.

### EXAMPLE 5: 3-[3-(3-Chloro-phenylamino)-imidazo[1,2-a]pyrazin-2-yl]-phenol (32)

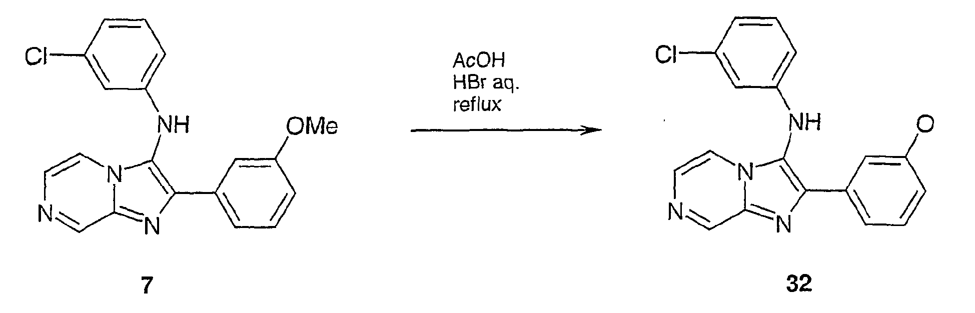

### Synthesis of 32 via methyl ether cleavage of 7

**7** was prepared in analogy to General Method **A** using 3-methoxybenzaldehyd. Pure **7** was obtained by crystallization from ethyl acetate / heptane.

**7** (50 mg, 0.14 mmol) were refluxed in a mixture of glacial acetic acid (0.5 mL) and aqueous hydrobromic acid (5 mL) for 20 hours. After cooling to rt, it was extracted with dichloromethane (3 x 100 mL). The combined organic layers were was washed with water, saturated sodium bicarbonate solution and water, then dried (Na₂SO₄) and evaporated. **32** was obtained as a slight yellow solid.

### EXAMPLE 6: Additional compounds

The following compounds shown in TABLE 1, TABLE 2, TABLE 3 and TABLE 4 were prepared in accordance with the methods provided in Examples 1 to 4. Those of ordinary skill in the art of organic synthesis will recognize when starting materials or reaction conditions should be varied to obtain the desired compound.

The analytical data of the compounds is summarized in TABLE 5

**TABLE 1, compounds synthesized according to General Procedure A**

| **Structure cpd** | | **Pyrazine** | **Aldehyde** | **Isonitrile** |
|---|---|---|---|---|
| 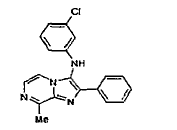 | **3** | **2** | Benzaldehyde | 3-Chlorophenylisonitrile |
| 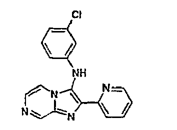 | **4** | 2-Amino-pyrazine | Pyridine-2-carbaldehyde | 3-Chlorophenylisonitrile |
| 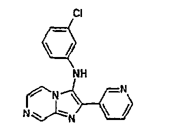 | **5** | 2-Amino-pyrazine | Pyridine-3-carbaldehyde | 3-Chlorophenylisonitrile |
| 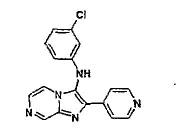 | **6** | 2-Amino-pyrazine | Pyridine-4-carbaldehyde | 3-Chlorophenylisonitrile |
| 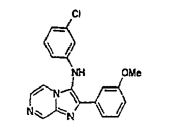 | **7** | 2-Amino-pyrazine | 3-Methoxybenzaldehyde | 3-Chlorophenylisonitrile |
| 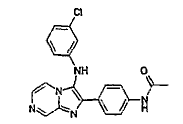 | **8** | 2-Amino-pyrazine | N-(4-Formylphenyl)-acetamide | 3-Chlorophenylisonitrile |
| 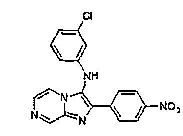 | **9** | 2-Amino-pyrazine | 4-Nitrobenzaldehyde | 3-Chlorophenylisonitrile |
| 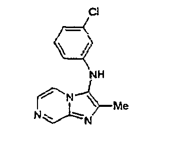 | **11** | 2-Amino-pyrazine | Acetaldehyde | 3-Chlorophenylisonitrile |
| 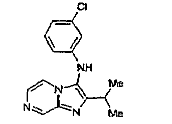 | **12** | 2-Amino-pyrazine | 2-Methylpropionaldehyde | 3-Chlorophenylisonitrile |
| 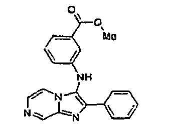 | **13** | 2-Amino-pyrazine | Benzaldehyde | 3-lsocyano-benzoic acid methyl ester |
| 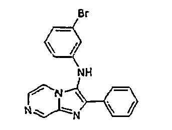 | **14** | 2-Amino-pyrazine | Benzaldehyde | 3-Bromophenylisonitrile |
| 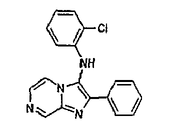 | **15** | 2-Amino-pyrazine | Benzaldehyde | 2-Chlorophenylisonitrile |
| 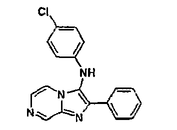 | **16** | 2-Amino-pyrazine | Benzaldehyde | 4-Chlorophenylisonitrile |
| 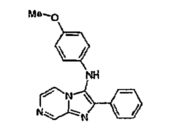 | **17** | 2-Amino-pyrazine | Benzaldehyde | 4-Methoxyphenylisonitrile |
| 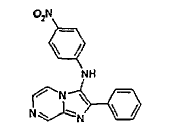 | **18** | 2-Amino-pyrazine | Benzaldehyde | 4-Nitrophenylisonitrile |
| 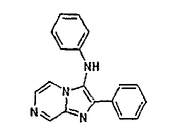 | **23** | 2-Amino-pyrazine | Benzaldehyde | Isocyanobenzene |
| 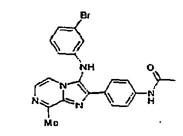 | **24** | **2** | N-(4-Formylphenyl)-acetamide | 3-Bromophenylisonitrile |
| 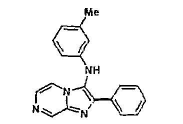 | **34** | 2-Amino-pyrazine | Benzaldehyde | 3-Methylphenylisonitrite |
| 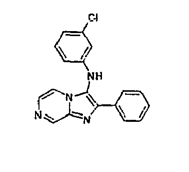 | **35** | 2-Amino-pyrazine | Benzaldehyde | 3-Chlorophenylisonitrile |
| 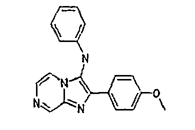 | **36** | 2-Amino-pyrazine | 4-Methoxybenzaldehyde | lsocyanobenzene |
| 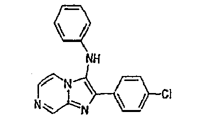 | **37** | 2-Amino-pyrazine | 4-Chlorobenzaldehyde | lsocyanobenzene |
| 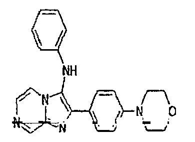 | **38** | 2-Amino-pyrazine | 4-Morpholino-benzaldehyde | Isocyanobenzene |
| 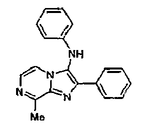 | **39** | **2** | Benzaldehyde | Isocyanobenzene |

**TABLE 2, compounds synthesized according to General Procedure B**

| **Structure** | **cpd** | **Pyrazine** | **Aldehyde** | **Isonitrile** |
|---|---|---|---|---|
| 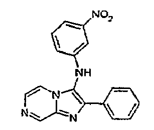 | **25** | 2-Amino-pyrazine | Benzaldehyde | 3-Nitro-phenylisonitrile |
| 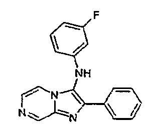 | **26** | 2-Amino-pyrazine | Benzaldehyde | 3-Fluorophenylisonitrile |
| 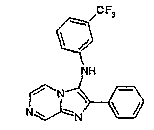 | **27** | 2-Amino-pyrazine | Benzaldehyde | 3-Trifluoromethyl-phenylisonitrile |
| 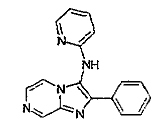 | **28** | 2-Amino-pyrazine | Benzaldehyde | 2-Isocyano-pyridine |
| 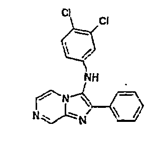 | **29** | 2-Amino-pyrazine | Benzaldehyde | 3,4-Di-chloro-phenylisonitrile |
| 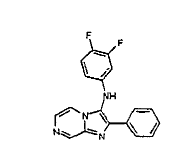 | **30** | 2-Amino-pyrazine | Benzaldehyde | 3,4-Di-fluorophenylisonitrile |
| 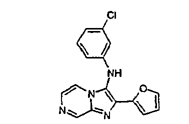 | **31** | 2-Amino-pyrazine | Furfualdehyde | 3-Chlorophenylisonitrile |
| 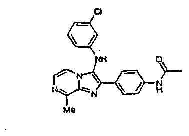 | **33** | **3** | N-(4-Formylphenyl)-acetamide | 3-Chlorophenylisonitrile |

**TABLE 3, compounds synthesized according to General Procedure C**

| **Structure** | **cpd** | **Pyrazine** | **Aldehyde** | **Isonitrile** | **Amine** |
|---|---|---|---|---|---|
| 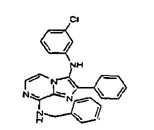 | **22** | 2-Amino-3-chloropyrazine | Benzaldehyde | 3-Chlorophenylisonitrile | Benzylamine |
| 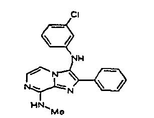 | **20** | 2-Amino-3-chloropyrazine | Benzaldehyde | 3-Chlorophenylisonitrile | Methylamine |
| 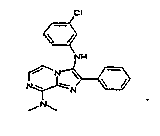 | **21** | 2-Amino-3-chloropyrazine | Benzaldehyde | 3-Chlorophenylisonitrile | Dimethylamine |

**TABLE 4, compounds synthesized according to General Procedure D**

| **Structure** | **cpd** | **Synthesis** |
|---|---|---|
| 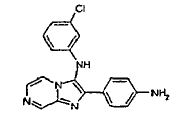 | **10** | from **9** |
| 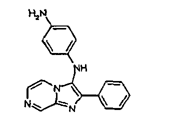 | **19** | from **18** |
| 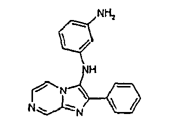 | **40** | from **25** |

**TABLE 5, analytical data**

| **Cpd** | **Name** | **Rt (HPLC) min.** | **Rt (LC-MS) min.** | **m/z [M+H]⁺** | **¹H NMR (300 MHz, DMSO) characteristic signals [ppm]** |
|---|---|---|---|---|---|
| **3** | (3-Chloro-phenyl)-(8-methyl-2-phenylimidazo[1,2-a]pyrazin-3-yl)-amine | 2.70 | 1.97 | 335 | 8.76, br, 1H; 7.82, d, 4.7 Hz, 1H; 2.87, s, 3H |
| **4** | (3-Chloro-phenyl)-(2-pyridin-2-yl-imidazo[1,2-a]pyrazin-3-yl)-amine | 2.02 | 1.72 | 322 | 9.14, d, 1.5 Hz; 8.75, s, 1H; 8.61, m, 1H |
| **5** | 3-Chloro-phenyl)-(2-pyridin-3-yl-imidazo[1,2-a]pyrazin-3-yl)-amine | 1.69 | 1.48 | 322 | 9.19, m, 2H; 8.73, s, 1H; 8.56, m, 1H |
| **6** | (3-Chloro-phenyl)-(2-pyridin-4-yl-imidazo[1,2-a]pyrazin-3-yl)-amine | 1.79 | 1.25 | 322 | 9.17, d, 1.4 Hz, 1H; 8.85, br, 1H; 6.80, m, 2H |
| **7** | (3-Chloro-phenyl)-[2-(3-methoxy-phenyl)-imidazo[1,2-a]pyrazin-3-yl]-amine | 2.68 | 1.92 | 351 | 9.13, d, 1.5 Hz, 1H; 8.67, s, 1H; 3.73, s, 3H |
| **8** | N-{4-[3-(3-Chlorophenylamino)-imidazo[1,2-a]pyrazin-2-yl]-phenyl}-acetamide | 2.24 | 1.63 | 378 | 10.1, br, 1H; 9.24, d, 1.4 Hz, 1H; 8.76, br, 1H; 2.06, s, 3H |
| **9** | (3-Chloro-phenyl)-[2-(4-nitro-phenyl)-imidazo[1,2-a]pyrazin-3-yl]-amine | 2.90 | 1.99 | 366 | 9.19, br, 1H; 8.82, br, 1H; 6.67, br, 1H |
| **10** | [2-(4-Amino-phenyl)-imidazo[1,2-a]pyrazin-3-yl]-(3-chloro-phenyl)-amine | 1.89 | 0.85 | 336 | 9.00, br, 1H; 8.51, br, 1H; 8.00, m, br, 1H |
| **11** | (3-Chloro-phenyl)-(2-methyl-imidazo[1,2-a]pyrazin-3-yl)-amine | 2.04 | 1.51 | 259 | 9.20, d, 1.1 Hz, 1 H; 8.60, br, 1H; 2.36, s, 3H |
| **12** | (3-Chloro-phenyl)-(2-isopropyl-imidazo[1,2-a]pyrazin-3-yl)-amine | 2.38 | 1.77 | 287 | 9.33, d, 0.8 Hz, 1H; 8.63, br, 1H; 1.34, d, 6.9 Hz, 6H |
| **13** | 3-(2-Phenyl-imidazo[1,2-a]pyrazin-3-ylamino)-benzoic acid methyl ester | 2.46 | 1.74 | 345 | 9.18, br, 1H; 8.76, br, 1H; 3.82, s, 3H |
| **14** | (3-Bromo-phenyl)-(2-phenyl-imidazo[1,2-a]pyrazin-3-yl)-amine | 2.81 | 1.94 | 365 | 9.22, d, 0.9 Hz; 8.74, br, 1H; 6.48, m, 1H |
| **15** | (2-Chloro-phenyl)-(2-phenyl-imidazo[1,2-a]pyrazin-3-yl)-amine | 2.79 | 1.94 | 321 | 9.14, d, 1.5 Hz, 1 H; 8.15, s, 1H; 6.08, m, 1H |
| **16** | (4-Chloro-phenyl)-(2-phenyl-imidazo[1,2-a]pyrazin-3-yl)-amine | 2.80 | 1.93 | 321 | 9.11, d, 1.4 Hz, 1H; 8.57, s, 1H; 6.54, m, 1H |
| **17** | (4-Methoxy-phenyl)-(2-phenyl-imidazo[1,2-a]pyrazin-3-yl)-amine | 2.41 | 1.70 | 317 | 9.09, d, 1.5 Hz, 1H; 5.76, s, 1H; 3.64, s, 3H |
| **18** | (4-Nitro-phenyl)-(2-phenyl-imidazo[1,2-alpyrazin-3-yl)-amine | 2.34 | 1.72 | 332 | 9.54, br, 1H; 9.19, br, 1H; 6.71, m, 2H |
| **19** | N-(2-Phenyl-imidazo[1,2-a]pyrazin-3-yl)-benzene-1,4-diamine | | 0.79 | 302 | |
| **20** | N'3-(3-Chloro-phenyl)-N'8-methyl-2-phenyl-imidazo[1,2-a]pyrazine-3,8-diamine | 2.49 | 1.64 | 350 | 8.44, br, 1H;7.90, m, 2H;2.92, d, 4.8 Hz, 3H |
| **21** | N'3-(3-Chloro-phenyl)-N'8,N'8-dimethyl-2-phenyl-imidazo[1,2-a]pyrazine-3,8-diamine | | 1.66 | 364 | |
| **22** | N'8-Benzyl-N'3-(3-chloro-phenyl)-2-phenyl-imidazo[1,2-a]pyrazine-3,8-diamine x HCl | 2.93 | 2.22 | 426 | 8.95, br, 1H;7.99, m, 2H;4.95, m,2H |
| **23** | Phenyl-(2-phenyl-imidazo[1,2-a]pyrazin-3-yl)-amine | 2.36 | 1.75 | 287 | 9.11, d, 1.4 Hz, 1 H; 8.41, br, 1H; 6.52, m, 2H |
| **24** | N-{4-[3-(3-Bromo-phenylamino)-8-methyl-imidazo[1,2-a]pyrazin-2-yl]-phenyl}-acetamide | 2.08 | 1.68 | 436 | 10.0, br, 1H; 8.59, br, 1H; 2.05, s, 3H |
| **25** | (3-Nitro-phenyl)-(2-phenyl-imidazo[1,2-a]pyrazin-3-yl)-amine | 2.35 | 1.75 | 332 | 9.15, d, 1.5 Hz, 1H; 9.03, s, 1H; 8.85, m, 1H: |
| **26** | (3-Fluoro-phenyl)-(2-phenyl-imidazo[1,2-a]pyrazin-3-yl)-amine | 2.36 | 1.81 | 305 | 9.23, d, 1.3 Hz, 1H; 8.76, br, 1H; 6.31, m, 2H |
| **27** | (2-Phenyl-imidazo[1,2-a]pyrazin-3-yl)-(3-trifluoromethyl-phenyl)-amine | 2.65 | 1.97 | 355 | 9.18, d, 1.5 Hz, 1H;8.88, s, 1H;6.71, m, 1H |
| **28** | (2-Phenyl-imidazo[1,2-a]pyrazin-3-yl)-pyridin-2-yl-amine | 1.41 | 1.29 | 288 | 9.11, d, 1.4 Hz, 1 H;8.06, m, 1H;8.80, m, 2H |
| **29** | (3,4-Dichloro-phenyl)-(2-phenyl-imidazo[1,2-a]pyrazin-3-yl)-amine | 2.76 | 2.03 | 355 | 9.15, d, 1.4 Hz, 1 H; 8.81, s, 1H; 6.50, m, 1 H |
| **30** | (3,4-Difluoro-phenyl)-(2-phenyl-imidazo[1,2-a]pyrazin-3-yl)-amine | 2.43 | 1.85 | 323 | 9.11, d, 1.5 Hz, 1H;8.58, s, 1H;6.58, m, 1H |
| **31** | (3-Chloro-phenyl)-(2-furan-2-yl-imidazo[1,2-a]pyrazin-3-yl)-amine | 2.31 | 1.74 | 311 | 9.08, d, 1.5 Hz, 1H;8.58, s, 1H;6.43, m, 1H |
| **32** | 3-[3-(3-Chloro-phenylamino)-imidazo[1,2-a]pyrazin-2-yl]-phenol | 2.22 | 1.70 | 337 | 9.32, br, 1H;8.86, br, 1H;6.50, m, 1H |
| **33** | N-{4-[3-(3-Chloro-phenylamino)-8-methyl-imidazo[1,2-a]pyrazin-2-yl]-phenyl}-acetamide | | 1.69 | 392 | |
| **34** | (2-Phenyl-imidazo[1,2-a]pyrazin-3-yl)-m-tolyl-amine | | 1.89 | 301 | 9.11, d, 1.5 Hz, 1H; 8.33, s, 1H; 2.16, s, 3H |
| **35** | (3-Chloro-phenyl)-(2-phenyl-imidazo[1,2-a]pyrazin-3-yl)-amine | | 1.92 | 321 | 9.12, d, 1.4 Hz, 1 H; 8.67, br, 1H; 6.42, m, 2H |
| **36** | [2-(4-Methoxy-phenyl)-imidazo[1,2-a]pyrazin-3-yl]-phenyl-amine | | 1.76 | 317 | 9.06, d, 1.5 Hz; 8.34, s, 1 H; 3.77, s, 3H |
| **37** | [2-(4-Chloro-phenyl)-imidazo[1,2-a]pyrazin-3-yl]-phenyl-amine | | 1.96 | 321 | 9.11, d, 1.4 Hz, 1H; 8.42, s, 1H; 6.51, d, 7.6 Hz, 2H |
| **38** | [2-(4-Morpholin-4-yl-phenyl)-imidazo[1,2-a]pyrazin-3-yl]-phenyl-amine | | 1.69 | 372 | 9.03, d, 1.5Hz, 1H; 3.72, m, 2H; 3.15, m, 2H |
| **39** | (8-Methyl-2-phenyl-imidazo[1,2-a]pyrazin-3-yl)-phenyl-amine | | 1.80 | 301 | 8.31, br, 1H; 8.00, m, 2H; 2.74, s, 3H |
| **40** | N-(2-Phenyl-imidazo[1,2-a]pyrazin-3-yl)-benzene-1,3-diamine | | 1.24 | 302 | |

### EXAMPLE 7: Assay for EPHB4 kinase activity

### Kinase assay protocol

The kinase inhibition activity of the compounds was measured in an in vitro kinase assay.

Briefly, in a final reaction volume of 25 µL, human EphB4 (N-terminal His6-tagged, recombinant, amino acids 561-end, expressed by baculovirus in Sf21 insect cells; 5-10 mU) was incubated with 8 mM MOPS pH 7.0, 0.2 mM EDTA, 10 mM MnCl₂, 0.1 mg/mL poly(Glu, Tyr) 4:1, 10 mM MgAcetate and [γ-³³P-ATP] (specific activity approx. 500 cpm/pmol, concentration as required). The reaction was initiated by the addition of the MgATP mix. After incubation for 40 min at rt, the reaction was stopped by the addition of 5 µL of a 3% phosphoric acid solution. 10 µL of the reaction was then spotted onto a Filtermat A and washed three times for 5 min in 75 mM phosphoric acid and once in methanol prior to drying and scintillation counting.

### EXAMPLE 8: Test results

All compounds described in Example 1 to 6 were tested in the assay for EPHB4 activity described in Example 7 and found to exhibit an IC₅₀ of 6 µM or less. Certain compounds disclosed in Example 1 to 6 exhibited an IC₅₀ of 500 nM or less in this assay. A subset of those compounds even exhibited an IC₅₀ of 150 nM or less.

While certain embodiments have been shown and described, numerous modifications and substitutions may be made without departing from the scope of the invention. Therefore, present invention has been described by way of examples and they have to be understood in an illustrative sense only and are not to be interpreted as limiting this invention in any manner.

### Enlarged structure formulas of the compounds of Tables 1 to 4

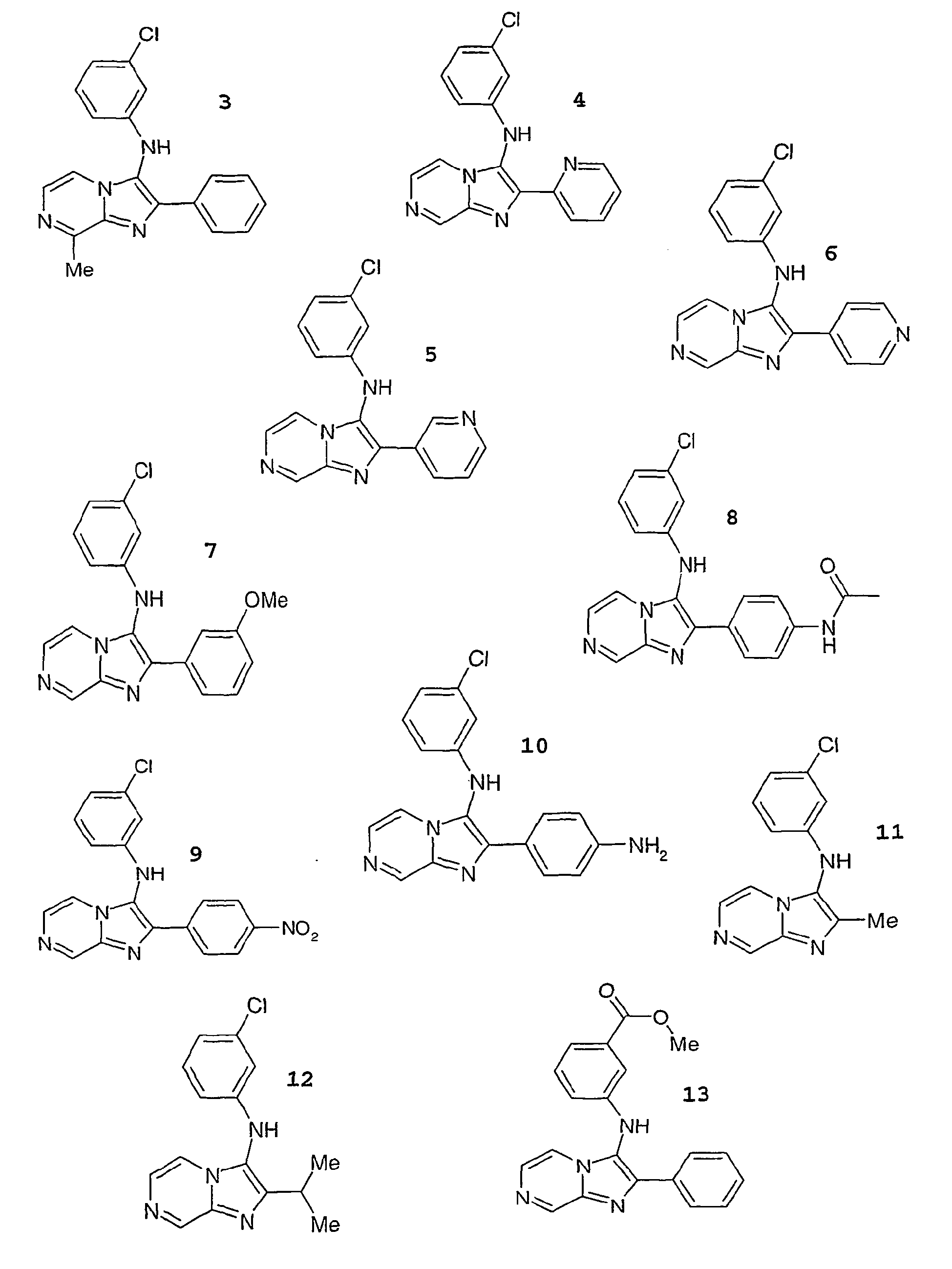

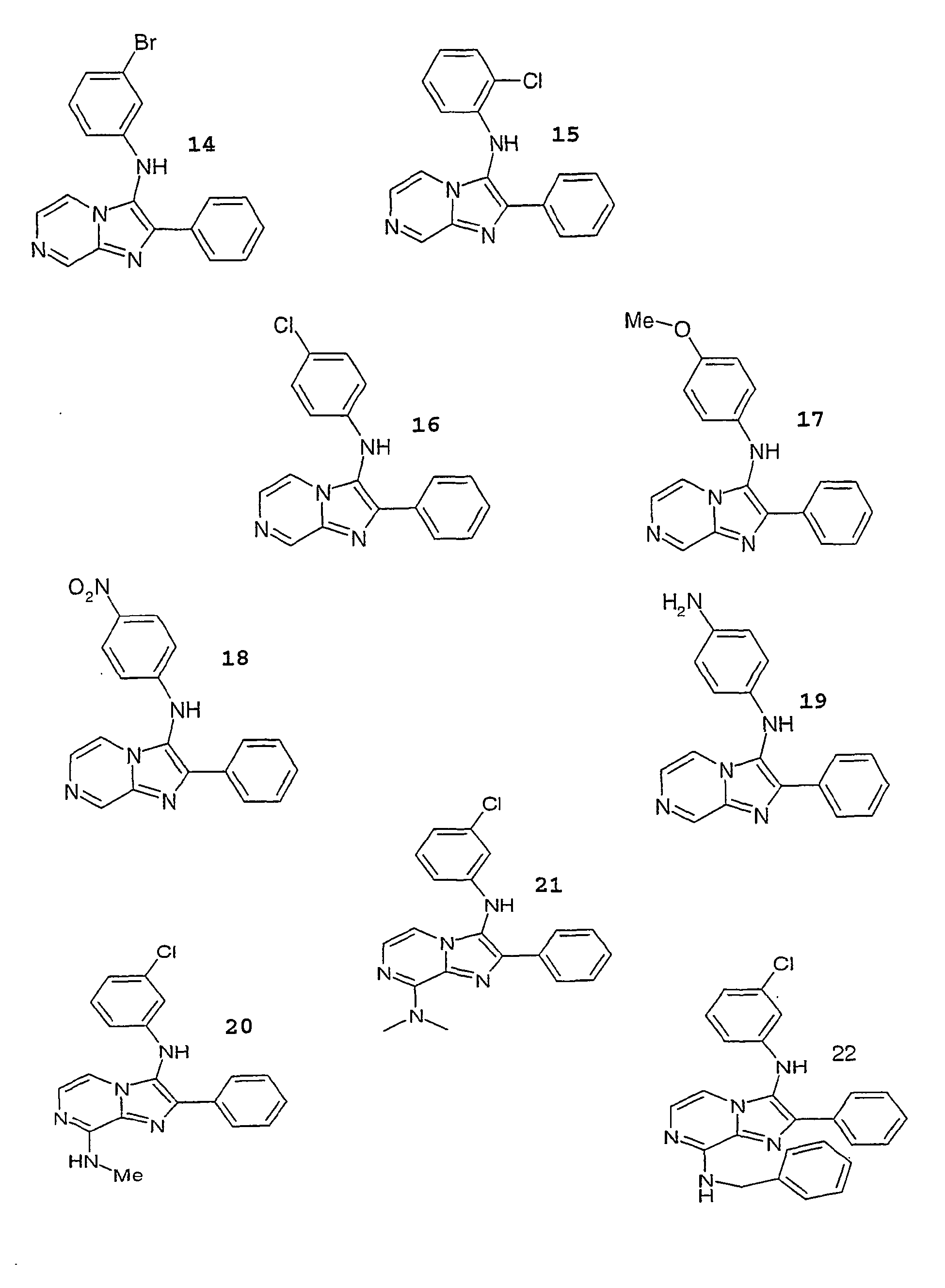

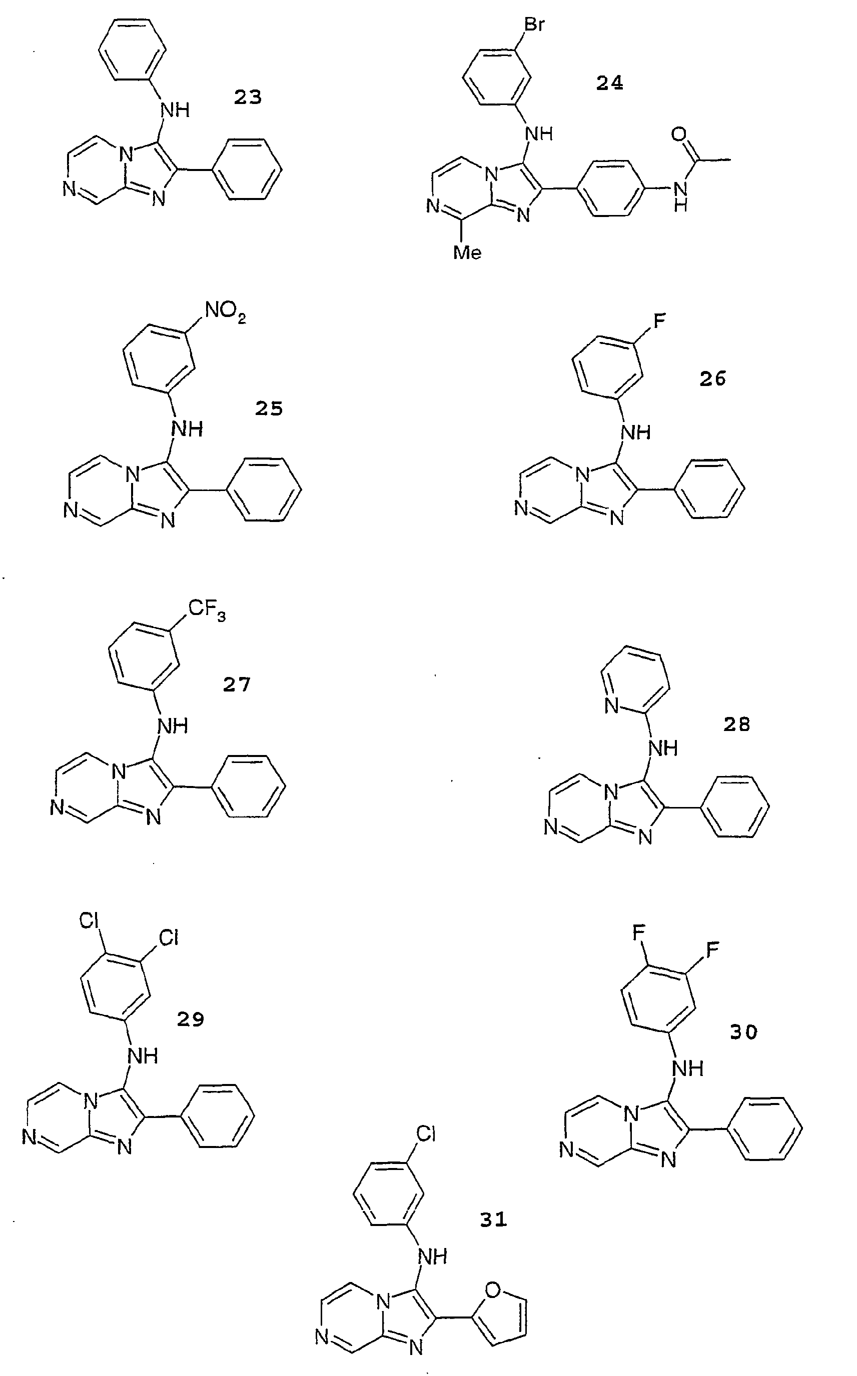

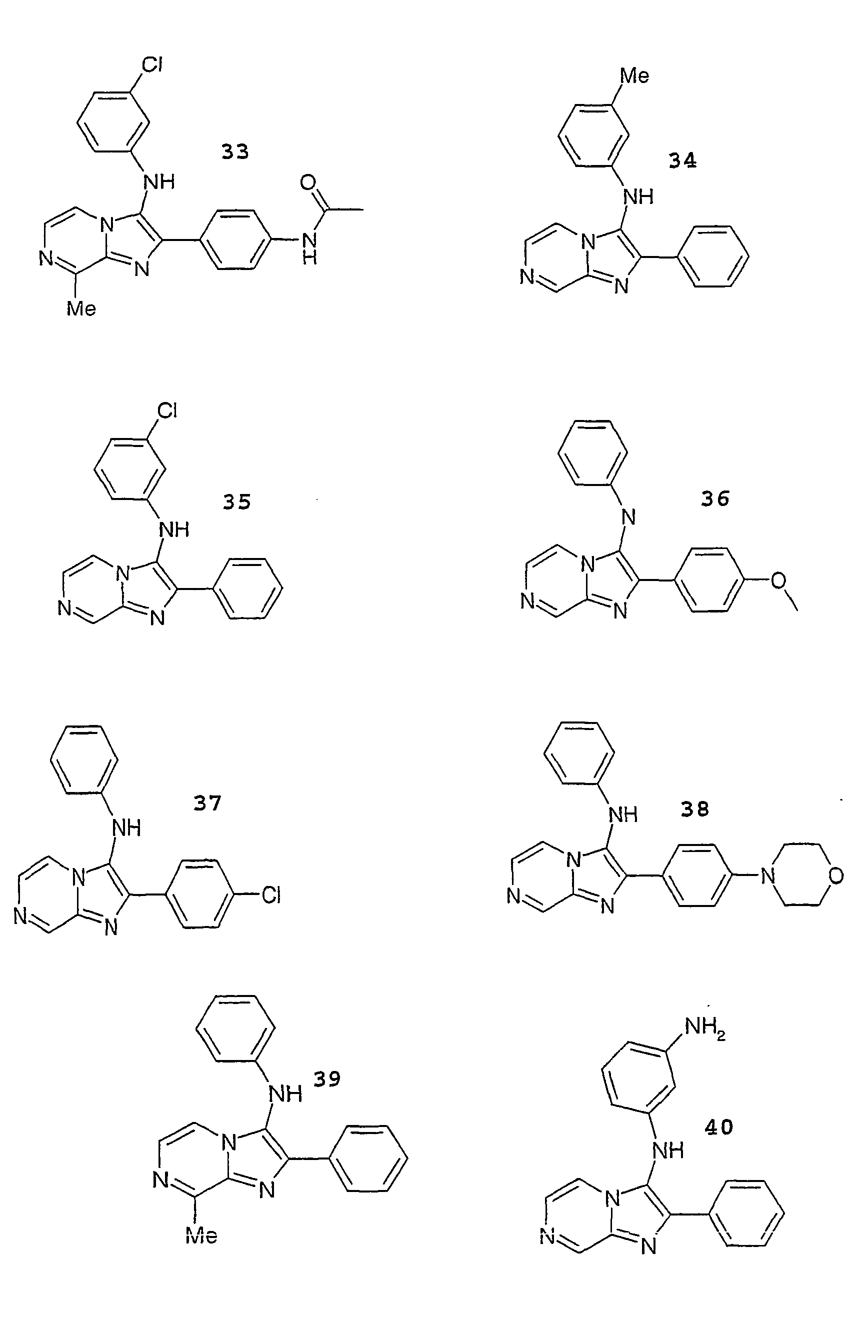

## Claims

1. Use of a compound of formula I 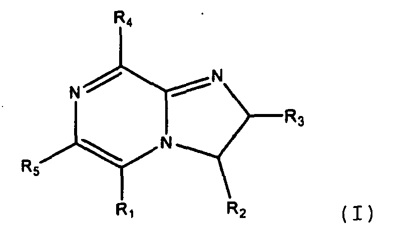 wherein
R₁, R₂ and R₄ are independently selected from hydrogen, optionally substituted C₁-C₆ alkyl, optionally substituted C₃-C₈ cycloalkyl, NR₆R₇, OR₆, SR₆, (CH) ₘR₆R₇, wherein R₆ and R₇ are independently selected from hydrogen, optionally substituted C₃-C₈ cycloalkyl, optionally substituted aryl, optionally substituted 5-or 6- membered heterocycle, (CH₂) ₙCO (O) R₈, (CH₂) ₘR' where n = 0, 1, 2, 3, 4 and wherein R₈ is hydrogen, C₁-C₆ alkyl, optionally substituted C₃-C₈ cycloalkyl, optionally substituted aryl and wherein R' is selected from hydrogen, C₁-C₆ alkyl, C₁-C₆ alkoxy, optionally substituted C₃-C₈ cycloalkyl, optionally substituted aryl, halogen, hydroxyl, NO₂, NH₂, SO₂NH₂ cyano and m is 0, 1, 2, 3, 4;
R₃ is hydroxyl, halogen, NH₂, NO₂, cyano, SH, optionally substituted C₁-C₆ alkyl, optionally substituted C₃-C₈ cycloalkyl, optionally substituted aryl, optionally substituted 5- or 6-membered heterocycle,
R₅ is selected from C₁-C₆ alkyl, hydrogen, hydroxyl, alkoxy, NH₂, halogen, cyano, alkine, COOR" wherein R" is selected from hydrogen, C₁-C₆ alkyl, C₃-C₈ cycloalkyl;
for the manufacture of a medicament for the treatment of a hyperproliferative disease or a malignant transformation involving a protein kinase.

2. The use of claim 1 wherein
R₁ is hydrogen, methyl, ethyl, propyl, cyclopropyl, NH₂, SH and
R₂ is NR₆R₇ wherein R₆ is hydrogen,
R₇ is selected from C₃-C₈ cycloalkyl, optionally substituted phenyl, (CH₂) ₙCO(O)R₈ where n = 1, and wherein R₈ is C₁-C₆ alkyl C₁-C₆ alkoxy,
R₃ is optionally substituted phenyl, optionally substituted 5- or 6-membered heterocyle, cyano;
R₄ is hydrogen, NH(CH₂) ₘR', O(CH₂) ₘR', S(CH₂) ₘR' wherein R' is selected from optionally substituted phenyl, optionally substituted C₃-C₈ cycloalkyl and m = 1, 2,
R₅ is methyl, hydrogen, hydroxyl.

3. The use of claim 1 or 2 wherein
R₁ is hydrogen,
R₂ is NHR₇ wherein R₇ is selected from C₃-C₈ cycloalkyl, phenyl optionally substituted with a substituent selected from C₁-C₆ alkyl, C₁-C₆ alkoxy, halogen, SO₂NH₂, hydroxyl; (CH₂) ₙCO(O)R₈ where n = 1, and wherein R₈ is methyl, ethyl, propyl, butyl,
R₃ is phenyl optionally substituted with a substituent selected from C₁-C₆ alkyl, C₁-C₆ alkoxy, halogen, hydroxyl, NO₂, NH₂, optionally substituted 5- or 6-membered heterocycle, cyano,
R₄ and R₅ are hydrogen.

4. The use of claims 1 to 3, wherein
R₁ is hydrogen,
R₂ is NHR₇ wherein R₇ is selected from phenyl optionally substituted with a radical selected from methyl, ethyl, propyl, halogen, methoxy, hydroxyl,
R₃ is phenyl optionally substituted with a substituent selected from methoxy, bromo, chloro, fluoro, hydroxyl, NO₂, NH₂, cyano, R₄ and R₅ are hydrogen.

5. The use of claims 1-4, wherein
R₁ is hydrogen,
R₂ is NHR₇ wherein R₇ is phenyl,
R₃ is phenyl and
R₄ and R₅ are hydrogen; and where
R₁ is hydrogen,
R₂ is NHR₇ wherein R₇ is phenyl,
R₃ is phenyl substituted with hydroxyl and
R₄ and R₅ are hydrogen.

6. The use of claim 1, wherein
R₁ is hydrogen,
R₂ is NR₆R₇ wherein R₆ is hydrogen, R₇ is optionally substituted phenyl,
R₃ is optionally substituted phenyl or optionally substituted, preferably unsubstituted, furyl
R₄ is hydrogen, methyl or NR₆R₇ wherein R₆ and R₇ are independently from each other selected from hydrogen, C₁-C₂ alkyl, optionally substituted by phenyl, and
R₅ is hydrogen.

7. The use of claim 6, wherein
R₁ is hydrogen,
R₂ is -NR₆R₇ wherein R₆ is hydrogen and R₇ is unsubstituted phenyl or phenyl substituted in 3 and/or 4 position by one or two substituents independently selected from halogen, C₁-C₂ alkoxy, in particular methoxy, C₁-C₂ alkyl, in particular methyl, CF₃, NO₂, NH₂, NHCH₃, N(CH₃) ₂,
R₃ is unsubstituted phenyl or phenyl substituted in 3 and/or 4 position by a substituent independently selected from C₁-C₄ alkoxy, or hydroxy, or amino, or the substituents in 3- and 4-position form together a 5- or 6-membered heterocycle,
R₄ is hydrogen or methyl and
R₅ is hydrogen.

8. The use of claim 7, wherein
R₁ is hydrogen,
R₂ is -NR₆R₇ wherein R₆ is hydrogen and R₇ is unsubstituted phenyl or phenyl substituted in 3 and/or 4 position by one or two substituents independently selected from halogen and CF₃, in particular an unsubstituted phenyl or a phenyl substituted in 3 or 4 position,
R₃ is unsubstituted phenyl or phenyl substituted in 3 and/or 4 position by methoxy or hydroxy, in particular an unsubstituted phenyl or a phenyl substituted in 3-or 4-position,
R₄ is hydrogen or methyl, and
R₅ is hydrogen.

9. The use of claim 8, wherein
R₁ is hydrogen,
R₂ is -NR₆R₇ wherein R₆ is hydrogen and R₇ is phenyl substituted with halogen in 3-position,
R₃ is unsubstituted phenyl,
R₄ is methyl, and
R₅ is hydrogen.

10. The use of claims 1 to 9, wherein said disease is cancer.

11. The use of claim 10, wherein said disease is selected from the group consisting of breast cancer, liver cancer, gastrointestinal cancer, neuroblastomas, leukemias and lymphomas, prostate cancer, pancreatic cancer, lung cancer, melanoma, ovarian cancer, thyroid cancers, sarcomas, renal carcinomas and epidermoid cancer.

12. The use of claims 1 to 11, wherein the disease is melanoma.

13. Use of a compound of formula Ia 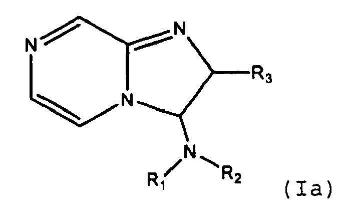 wherein
R₁ is C₃-C₈ cycloalkyl, phenyl optionally substituted with a substituent selected from C₁-C₆ alkyl, SO₂NH₂, halogen, C₁-C₆ alkoxy; (CH₂) ₙCO(O)R₄ where n = 1, 2, 3, 4 and wherein R₄ is hydrogen, optionally substituted C₁-C₆ alkyl, optionally substituted C₃-C₈ cycloalkyl, optionally substituted aryl,
R₂ is hydrogen, C₁-C₆ alkyl and
R₃ is phenyl optionally substituted with a substituent selected from C₁-C₆ alkyl, C₁-C₆ alkoxy, halogen, hydroxyl, NO₂
for the manufacture of a medicament for the treatment of a hyperproliferative disease or a malignant transformation involving a protein kinase.

14. The use of claim 13 wherein
R₁ is cyclopentyl, cyclohexyl, phenyl optionally substituted with a substituent selected from methyl, ethyl, propyl; (CH₂) ₙCO(O)R₄ where n = 1 and wherein R₄ is methyl, ethyl, propyl,
R₂ is hydrogen and
R₃ is phenyl optionally substituted with a substituent selected from methoxy, ethoxy, hydroxyl, NO₂, bromine, fluorine, chlorine.

15. The use of claim 13 or 14, wherein
R₁ is phenyl, R₂ is hydrogen and R₃ is phenyl substituted with hydroxyl and
R₁ is phenyl, R₂ is hydrogen and R₃ is phenyl.

16. A compound of formula Ib as medicament 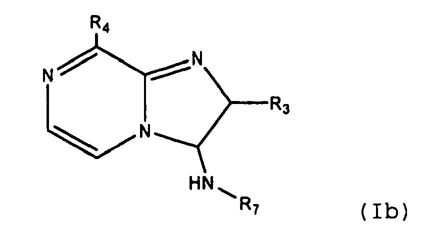
R₇ is optionally substituted phenyl,
R₃ is optionally substituted phenyl or optionally substituted, preferably unsubstituted, furyl, and
R₄ is methyl or NR6R7 wherein R6 and R₇ are independently from each other selected from hydrogen, C₁-C₂ alkyl, optionally substituted by phenyl.

17. The compound of claim 16, wherein
R₇ is unsubstituted phenyl or phenyl substituted in 3 and/or 4 position by one or two substituents independently selected from halogen, C₁-C₂ alkoxy, in particular methoxy, C₁-C₂ alkyl, in particular methyl, CF₃, NO₂, NH₂, NHCH₃, N(CH₃)₂,
R₃ is unsubstituted phenyl or phenyl substituted in 3 and/or 4 position by a substituent independently selected from C₁-C₄ alkoxy, or hydroxy, or amino, or the substituents in 3- and 4-position form together a 5- or 6-membered heterocycle, and
R₄ is methyl.

18. The compound of claim 17, wherein
R₇ is unsubstituted phenyl or phenyl substituted in 3 and/or 4 position by one or two substituents independently selected from halogen, and CF₃, in particular an unsubstituted phenyl or a phenyl substituted in 3 or 4 position,
R₃ is unsubstituted phenyl or phenyl substituted in 3 and/or 4 position by methoxy or hydroxy, in particular an unsubstituted phenyl or a phenyl substituted in 3-or 4-position, and
R₄ is methyl.

19. The compound of claim 18, wherein
R₇ is phenyl substituted with halogen in 3-position,
R₃ is unsubstituted phenyl, and
R₄ is methyl.

20. A compound of the formula 3, 24, 33.

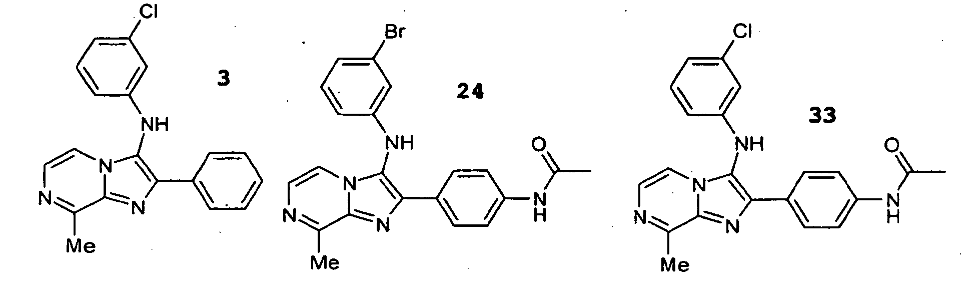

## Patentansprüche

1. Verwendung einer Verbindung der Formel (I) 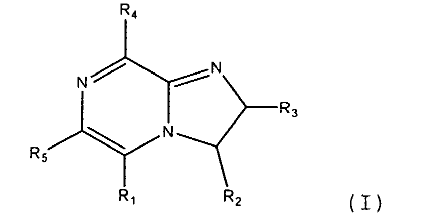 worin
R₁, R₂ und R₄ unabhängig voneinander ausgewählt sind aus Wasserstoff, gegebenenfalls substituiertem C₁-C₆ Alkyl, gegebenenfalls substituiertem C₃-C₈ Cycloalkyl, NR₆R₇, OR₆, SR₆, (CH)ₘR₆R₇, (CH₂)ₙCO(O)R₈, (CH₂)ₘR' worin
R₆ und R₇ unabhängig voneinander ausgewählt sind aus Wasserstoff, gegebenenfalls substituiertem C₃-C₈ Cycloalkyl, gegebenenfalls substituiertem Aryl, gegebenenfalls substituiertem 5- oder 6-gliedrigem Heterocyclus,
R₈ Wasserstoff, C₁-C₆ Alkyl, gegebenenfalls substituiertem C₃-C₈ Cycloalkyl, gegebenenfalls substituiertem Aryl ist,
R' ausgewählt ist aus Wasserstoff, C₁-C₆ Alkyl, C₁-C₆ Alkoxy, gegebenenfalls substituiertem C₃-C₈ Cycloalkyl, gegebenenfalls substituiertem Aryl, Halogen, Hydroxyl, NO₂ NH₂, SO₂NH₂, Cyano,
m = 0, 1, 2, 3, 4 ist, und
n = 0, 1, 2, 3, 4 ist;
R₃ Hydroxyl, Halogen, NH₂, NO₂, Cyano, SH, gegebenenfalls substituiertes C₁-C₆ Alkyl, gegebenenfalls substituiertes C₃-C₈ Cycloalkyl, gegebenenfalls substituiertes Aryl, gegebenenfalls substituierter 5- oder 6-gliedriger Heterocyclus ist;
R₅ ausgewählt ist aus C₁-C₆ Alkyl, Wasserstoff, Hydroxyl, Alkoxy, NH₂, Halogen, Cyano, Alkin, COOR" worin R'' ausgewählt ist aus Wasserstoff, C₁-C₆ Alkyl, C₃-C₈ Cycloalkyl;
zur Herstellung eines Medikamentes zur Behandlung einer hyperproliferativen Erkrankung oder einer krankhaften Veränderung welche mit einer Proteinkinase verknüpft ist.

2. Die Verwendung von Anspruch 1 worin
R₁ Wasserstoff, Methyl, Ethyl, Propyl, Cyclopropyl, NH₂, SH ist;
R₂ NR₆R₇ ist, worin
R₆ Wasserstoff ist,
R₇ ausgewählt ist aus C₃-C₈ Cycloalkyl, gegebenenfalls substituiertes Phenyl, (CH₂)ₙCO(O)R₈ worin n = 1 und worin R₈ C₁-C₆ Alkyl, C₁-C₆ Alkoxy ist;
R₃ gegebenenfalls substituiertes Phenyl, gegebenenfalls substituierter 5- oder 6-gliedriger Heterocyclus, Cyano ist;
R₄ Wasserstoff, NH(CH₂)ₘR', O(CH₂)ₘR', S(CH₂)ₘR' ist, worin R' ausgewählt ist aus gegebenenfalls substituiertes Phenyl, gegebenenfalls substituiertes C₃-C₈ Cycloalkyl und m = 1, 2 ist;
R₅ Methyl, Wasserstoff, Hydroxyl ist.

3. Die Verwendung gemäss Anspruch 1 oder 2 worin
R₁ Wasserstoff ist;
R₂ NHR₇ ist, worin R₇ ausgewählt ist aus C₃-C₈ Cycloalkyl; Phenyl gegebenenfalls substituiert durch einen Substituenten ausgewählt aus C₁-C₆ Alkyl, C₁-C₆ Alkoxy, Halogen, SO₂NH₂, Hydroxyl; (CH₂)ₙCO(O)R₈ worin n = 1 und R₈ Methyl, Ethyl, Propyl, Butyl ist;
R₃ Phenyl ist, welches gegebenenfalls substituiert ist durch einen Substituenten ausgewählt aus C₁-C₆ Alkyl, C₁-C₆ Alkoxy, Halogen, Hydroxyl, NO₂, NH₂, gegebenenfalls substituierten 5- oder 6-gliedrigen Heterocyclus, Cyano;
R₄ und R₅ Wasserstoff sind.

4. Die Verwendung gemäss der Ansprüche 1 bis 3, worin
R₁ Wasserstoff ist;
R₂ NHR₇ ist, worin R₇ ausgewählt ist aus Phenyl gegebenenfalls substituiert durch ein Radikal ausgewählt aus Methyl, Ethyl, Propyl, Halogen, Methoxy, Hydroxyl;
R₃ Phenyl ist, gegebenenfalls substituiert durch einen Substituenten ausgewählt aus Methoxy, Bromo, Chloro, Fluoro, Hydroxyl, NO₂, NH₂, Cyano;
R₄ und R₅ Wasserstoff sind.

5. Die Verwendung gemäss der Ansprüche 1 bis 4, worin
R₁ Wasserstoff ist;
R₂ NHR₇ ist, worin R₇ Phenyl ist;
R₃ Phenyl ist und
R₄ und R₅ Wasserstoff sind;
und worin
R₁ Wasserstoff ist;
R₂ NHR₇ ist, worin R₇ Phenyl ist;
R₃ Phenyl ist welches durch Hydroxyl substituiert ist und
R₄ und R₅ Wasserstoff sind.

6. Die Verwendung gemäss Anspruch 1, worin
R₁ Wasserstoff ist;
R₂ NR₆R₇ ist, worin R₆ Wasserstoff ist, R₇ gegebenenfalls substituiertes Phenyl ist;
R₃ gegebenenfalls substituiertes Phenyl oder gegebenenfalls substituiertes, bevorzugt unsubstituiertes, Furyl ist;
R₄ Wasserstoff, Methyl oder NR₆R₇ ist, worin R₆ und R₇ unabhängig voneinander ausgewählt sind aus Wasserstoff, C₁-C₂ Alkyl, gegebenenfalls substituiert durch Phenyl und
R₅ Wasserstoff ist.

7. Die Verwendung gemäss Anspruch 6, worin
R₁ Wasserstoff ist;
R₂ -NR₆R₇ ist, worin R₆ Wasserstoff ist und R₇ unsubstituiertes Phenyl ist oder substituiertes Phenyl ist welches in 3 und/oder 4 Position durch einen oder zwei Substituenten unabhängig voneinander ausgewählt aus Halogen, C₁-C₂ Alkoxy (insbesondere Methoxy) C₁-C₂ Alkyl (insbesondere Methyl) CF₃, NO₂, NH₂, NHCH₃, N(CH₃)₂ substituiert ist;
R₃ unsubstituiertes Phenyl ist oder substituiertes Phenyl ist welches in 3 und/oder 4 Position durch einen Substituenten unabhängig voneinander ausgewählt aus C₁-C₄ Alkoxy oder Hydroxy oder Amino oder die Substituenten in 3- und 4-Position bilden gemeinsam einen 5- oder 6-gliedrigen Heterocyclus;
R₄ Wasserstoff oder Methyl ist;
R₅ Wasserstoff ist.

8. Die Verwendung gemäss Anspruch 7, worin
R₁ Wasserstoff ist;
R₂ -NR₆R₇ ist, worin R₆ Wasserstoff ist und R₇ unsubstituiertes Phenyl ist oder substituiertes Phenyl ist, welches in 3 und/oder 4 Position durch einen oder zwei Substituenten unabhängig voneinander ausgewählt aus Halogen und CF₃ ist, insbesondere unsubstituiertes Phenyl oder Phenyl substituiert in 3 oder 4 Position;
R₃ unsubstituiertes Phenyl ist oder substituiertes Phenyl ist welches in 3 und/oder 4 Position durch Methoxy oder Hydroxy substituiert ist, insbesondere unsubstituiertes Phenyl oder Phenyl substituiert in 3- oder 4-Position;
R₄ Wasserstoff oder Methyl ist und
R₅ Wasserstoff ist.

9. Die Verwendung qemäss Anspruch 8, worin
R₁ Wasserstoff ist;
R₂ -NR₆R₇ ist, worin R₆ Wasserstoff ist und R₇ in 3-Postion durch Halogen substituiertes Phenyl ist;
R₃ unsubstituiertes Phenyl ist;
R₄ Methyl ist und
R₅ Wasserstoff ist.

10. Die Verwendung gemäss Ansprüchen 1 bis 9, wobei besagte Krankheit Krebs ist.

11. Die Verwendung gemäss Anspruch 10, wobei besagte Krankheit ausgewählt ist aus der Gruppe umfassend Brustkrebs, Leberkrebs, Magen-Darm-Krebs, Neuroblastom, Leukämie und Lymphoma, Prostata Krebs, Bauchspeicheldrüsenkrebs, Lungenkrebs, Melanoma, Eierstock-Krebs, Schilddrüsen-Krebs, Sarcoma, Krebsgeschwüren der Niere und epidermoider Krebs.

12. Die Verwendung gemäss Ansprüchen 1 bis 11, wobei die Krankheit ein Melanom ist.

13. Die Verwendung einer Verbindung der Formel (Ia) 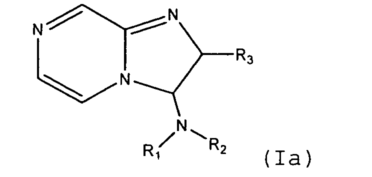 worin
R₁ C₃-C₈ Cycloalkyl, Phenyl gegebenenfalls substituiert durch einen Substituenten ausgewählt aus C₁-C₆ Alkyl, SO₂NH₂, Halogen, C₁-C₆ Alkoxy; (CH₂)ₙCO(O)R₄ worin n = 1, 2, 3, 4 und R₄ Wasserstoff ist, gegebenenfalls substituiertes C₁-C₆ Alkyl, gegebenenfalls substituiertes C₃-C₈ Cycloalkyl, gegebenenfalls substituiertes Aryl ist;
R₂ Wasserstoff, C₁-C₆ Alkyl ist und
R₃ Phenyl ist, gegebenenfalls substituiert durch einen Substituenten ausgewählt aus C₁-C₆ Alkyl, C₁-C₆ Alkoxy, Halogen, Hydroxyl, NO₂;
zur Herstellung eines Medikamentes zur Behandlung einer hyperproliferativen Erkrankung oder einer krankhaften Veränderung welche mit einer Proteinkinase verknüpft ist.

14. Die Verwendung gemäss Anspruch 13 worin
R₁ Cyclopentyl, Cyclohexyl, Phenyl gegebenenfalls substituiert durch einen Substituenten ausgewählt aus Methyl, Ethyl, Propyl; (CH₂)ₙCO(O)R₄ worin n für 1 und R₄ für Methyl, Ethyl, Propyl steht, ist;
R₂ Wasserstoff ist und
R₃ Phenyl, welches gegebenenfalls substituiert ist durch einen Substituent ausgewählt aus Methoxy, Ethoxy, Hydroxyl, NO₂ Bromo, Fluoro, Chloro, ist.

15. Die Verwendung gemäss Anspruch 13 oder 14 worin
R₁ Phenyl ist,
R₂ Wasserstoff ist und
R₃ Phenyl substituiert mit Hydroxyl ist
und
R₁ Phenyl ist,
R₂ Wasserstoff ist und
R₃ Phenyl ist.

16. Eine Verbindung der Formel (Ib) als Medikament 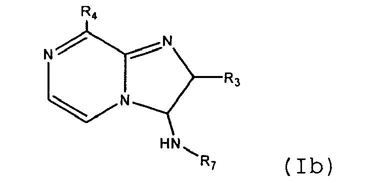 worin
R₇ gegebenenfalls substituiertes Phenyl ist,
R₃ gegebenenfalls substituiertes Phenyl oder gegebenenfalls substituiertes, bevorzugt unsubstituiertes, Furyl ist und
R₄ Methyl oder NR₆R₇ ist, worin R₆ und R₇ unabhängig voneinander ausgewählt sind aus Wasserstoff, C₁-C₂ Alkyl, gegebenenfalls substituiert durch Phenyl.

17. Die Verbindung gemäss Anspruch 16, worin
R₇ unsubstituiertes Phenyl ist oder Phenyl ist substituiert in 3 und/oder 4 Position durch einen oder zwei Substituenten unabhängig ausgewählt aus Halogen, C₁-C₂ Alkoxy (insbesondere Methoxy), C₁-C₂ Alkyl (insbesondere Methyl) CF₃, NO₂, NH₂, NHCH₃, N(CH₃)₂,;
R₃ unsubstituiertes Phenyl ist oder Phenyl ist substituiert in 3 und/oder 4 Position durch einen Substituenten unabhängig ausgewählt aus C₁-C₄ Alkoxy, oder Hydroxy, oder Amino, oder die Substituenten in 3-und 4-Position bilden gemeinsam einen 5- oder 6-gliedrigen Heterocyclus, und
R₄ Methyl ist.

18. Die Verbindung gemäss Anspruch 17, worin
R₇ unsubstituiertes Phenyl ist oder Phenyl ist substituiert in 3 und/oder 4 Position durch einen oder zwei Substituenten unabhängig ausgewählt aus Halogen und CF₃, insbesondere ein unsubstituiertes Phenyl oder ein Phenyl substituiert in 3 oder 4 Position;
R₃ unsubstituiertes Phenyl ist oder Phenyl ist substituiert in 3 und/oder 4 Position durch Methoxy oder Hydroxy, insbesondere ein unsubstituiertes Phenyl oder ein Phenyl substituiert in 3- oder 4-Position, und
R₄ Methyl ist.

19. Die Verbindung gemäss Anspruch 18, worin
R₇ durch Halogen in 3-Position substituiertes Phenyl ist;
R₃ unsubstituiertes Phenyl ist und
R₄ Methyl ist.

20. Eine Verbindung der Formel 3, 24, 33:

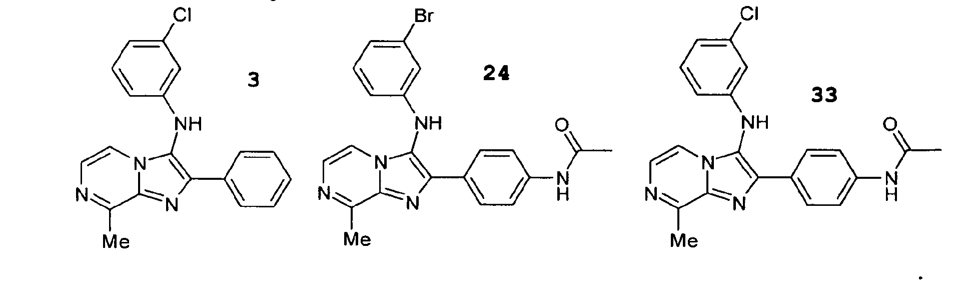

## Revendications

1. Utilisation d'un composé de formule I 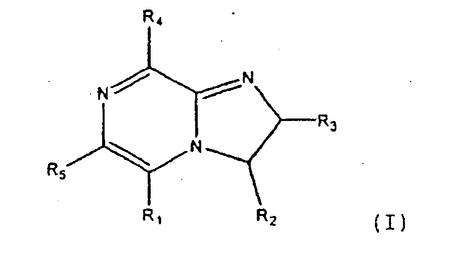 dans laquelle
R₁, R₂ et R₄ sont choisis indépendamment parmi hydrogène, alcoyle ayant de 1 à 6 atomes de carbone, éventuellement substitué, cycloalcoyle ayant de 3 à 8 atomes de carbone, éventuellement substitué, NR₆R₇, OR₆, SR₆, (CH)ₘR₆R₇, dans laquelle R₆ et R₇ sont choisis indépendamment parmi hydrogène, cycloalcoyle ayant de 3 à 8 atomes de carbone, éventuellement substitué, aryle éventuellement substitué, hétérocycle à 5 ou 6 chaînons, éventuellement substitué, (CH₂)ₙCO(0) R₈, (CH₂)ₘR' dans lesquelles n= 0, 1, 2, 3, 4 et dans lesquelles R₈ est hydrogène, alcoyle ayant de 1 à 6 atomes de carbone, cycloalcoyle ayant de 3 à 8 atomes de carbone, éventuellement substitué, aryle, éventuellement substitué, et dans lesquelles R' est choisi parmi hydrogène, alcoyle ayant de 1 à 6 atomes de carbone, alkoxy ayant de 1 à 6 atomes de carbone, cycloalcoyle ayant de 3 à 8 atomes de carbone, éventuellement substitué, aryle, éventuellement substitué, halogène, hydroxyle, NO₂, NH₂, SO₂NH₂, cyano et m est 0, 1, 2, 3, 4 ;
R₃ est hydroxyle, halogène, NH₂, NO₂, cyano, SH, alcoyle ayant de 1 à 6 atomes de carbone, éventuellement substitué, cycloalcoyle ayant de 3 à 8 atomes de carbone, éventuellement substitué, aryle, éventuellement substitué, hétérocycle à 5 ou 6 chaînons éventuellement substitué,
R₅ est choisi parmi alcoyle ayant de 1 à 6 atomes de carbone, hydrogène, hydroxyle, alcoxy, NH₂, halogène, cyano, alcyne, COOR", R" étant choisi parmi l'hydrogène, alcoyle ayant de 1 à 6 atomes de carbone, cycloalcoyle ayant de 3 à 8 atomes de carbone ;
pour la fabrication d'un médicament pour le traitement d'une maladie hyperproliférative ou d'une transformation maligne impliquant une protéine kinase.

2. Utilisation suivant la revendication 1, dans laquelle
R₁ est hydrogène, méthyle, éthyle, propyle, cyclopropyle, NH₂, SH et
R₂ est NR₆R₇ dans laquelle R₆ est hydrogène,
R₇ est choisi parmi cycloalcoyle ayant de 3 à 8 atomes de carbone, phényle éventuellement substitué, (CH₂)ₙCO(O)R₈ dans laquelle n = 1, et dans laquelle R₈ et alcoyle ayant de 1 à 6 atomes de carbone, alcoxy ayant de 1 à 6 atomes de carbone,
R₃ est phényle, éventuellement substitué, hétérocycle à 5 ou 6 chaînons, éventuellement substitué, cyano ;
R₄ est hydrogène, NH(CH₂)ₘR', O(CH₂)ₘR', S(CH₂)ₘR' , dans lesquelles R' est choisi parmi phényle éventuellement substitué, cycloalcoyle ayant de 3 à 8 atomes de carbone, éventuellement substitué et m = 1, 2,
R₅ est méthyle, hydrogène, hydroxyle.

3. Utilisation selon la revendication 1 ou 2, dans laquelle
R₁ est hydrogène,
R₂ est NHR₇ dans laquelle R₇ est choisi parmi cycloalcoyle ayant de 3 à 8 atomes de carbone, phényle, éventuellement substitué par un substituant choisi parmi alcoyle ayant de 1 à 6 atomes de carbone, alcoxy ayant de 1 à 6 atomes de carbone halogène , SO₂NH₂, hydroxyle ; (CH₂)ₙCO(O)R₈ dans laquelle n = 1, et dans laquelle R₈ est méthyle, éthyle, propyle, butyle,
R₃ est phényle, éventuellement substitué par un substituant choisi parmi alcoyle ayant de 1 à 6 atomes de carbone, alcoxy ayant de C₁-C₆ atome de carbone, halogène, hydroxyle, NO₂, NH₂, hétérocycle à 5 ou 6 chaînons, éventuellement substitué, cyano,
R₄ et R₅ sont hydrogène.

4. Utilisation selon les revendications 1 à 3, dans laquelle
R₁ est hydrogène,
R₂ est NHR₇ dans laquelle R₇ est choisi parmi phényle, éventuellement substitué par un radical choisi parmi méthyle, éthyle, propyle, halogène, méthoxy, hydroxyle,
R₃ est phényle, éventuellement substitué par un substituant choisi parmi méthoxy, bromo, chloro, fluoro, hydroxyle, NO₂, NH₂, cyano, R₄ et R₅ sont hydrogène.

5. Utilisation selon les revendications 1 à 4, dans laquelle
R₁ est hydrogène,
R₂ est NHR₇ dans laquelle R₇ est phényle,
R₃ est phényle et
R₄ et R₅ sont hydrogène ; et dans laquelle
R₁ est hydrogène,
R₂ est NHR₇ dans laquelle R₇ est phényle,
R₃ est phényle substitué par hydroxyle et
R₄ et R₅ sont hydrogène.

6. Utilisation selon la revendication 1, dans laquelle
R₁ est hydrogène,
R₂ est NR₆R₇ dans laquelle R₆ est hydrogène, R₇ est phényle, éventuellement substitué,
R₃ est phényle éventuellement substitué, ou furyle, éventuellement substitué, de préférence non substitué
R₄ est hydrogène, méthyle ou NR₆R₇ dans laquelle R₆ et R₇ sont indépendamment l'un de l'autre choisis parmi hydrogène, alcoyle ayant 1 ou 2 atome de carbone, phényle, éventuellement substitué, et
R₅ est hydrogène.

7. Utilisation selon la revendication 6, dans laquelle
R₁ est hydrogène,
R₂ est NR₆R₇ dans laquelle R₆ est hydrogène, R₇ est phényle non substitué ou phényle substitué en la position 3 et/ou en la position 4 par un ou par deux substituants choisi indépendamment parmi halogène, alcoxy ayant 1 ou 2 atome de carbone, en particulier méthoxy, alcoxy ayant 1 à 2 atome de carbone, en particulier méthyle, CF₃, NO₂, NH₂, NHCH₃, N(CH₃)₂,
R₃ est phényle non substitué ou phényle substitué en la position 3 et/ou en la position 4 par un substituant choisi indépendamment parmi alcoxy ayant de 1 à 4 atomes de carbone, ou hydroxy, ou amino, ou les substituants en les positions 3 et 4 forment ensemble un hétérocycle à 5 ou 6 chaînons,
R₄ est hydrogène ou méthyle et
R₅ est hydrogène.

8. Utilisation selon la revendication 7 dans laquelle
R₁ est hydrogène,
R₂ est NR₆R₇ dans laquelle R₆ est hydrogène et R₇ est phényle non substitué ou phényle substitué en la position 3 et/ou en la position 4 par un ou par deux substituants choisi indépendamment parmi halogène et CF₃, en particulier un phényle non substitué ou un phényle substitué en la position 3 ou 4,
R₃ est phényle non substitué ou phényle substitué en la position 3 et/ou en la position 4 par méthoxy ou hydroxy, en particulier un phényle non substitué ou un phényle substitué en la position 3 ou en la position 4,
R₄ est hydrogène ou méthyle et
R₅ est hydrogène.

9. Utilisation selon la revendication 9 dans laquelle
R₁ est hydrogène,
R₂ est NR₆R₇ dans laquelle R₆ est hydrogène et R₇ est phényle substitué par halogène en la position 3,
R₃ est phényle non substitué,
R₄ est méthyle, et
R₅ est hydrogène.

10. Utilisation suivant les revendications 1 à 9, dans laquelle la maladie est le cancer.

11. Utilisation suivant la revendication 10, dans lequel le cancer est choisi dans le groupe constitant en le cancer du sein, le cancer du foie, le cancer gastro-intestinal, les neuroblasmes, les leucémies et les lymphomes, le cancer de la prostate, le cancer du pancréas, le cancer du poumon, les mélanomes, le cancer de l'ovaire, les cancers de la thyroïde, les sarcomes, les carcinomes rénaux et le cancer épidermoïde.

12. Utilisation suivant les revendications 1 à 11, dans laquelle la maladie est un mélanome.

13. Utilisation d'un composé de formule Ia 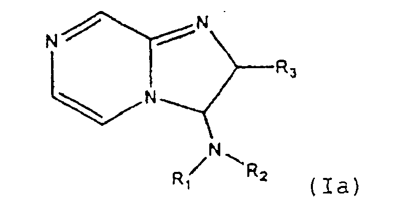 dans laquelle
R₁ est cycloalcoyle ayant de 1 à 8 atomes de carbone, phényle, éventuellement substitué par un substituant choisi parmi alcoyle ayant de 1 à 6 atomes de carbone, SO₂NH₂, halogène, alcoxy ayant de 1 à 6 atomes de carbone ; (CH₂)ₙCO(O)R₄ dans laquelle n = 1, 2, 3, 4 et dans laquelle R₄ est hydrogène, alcoyle ayant de 1 à 6 atomes de carbone, éventuellement substitué, cycloalcoyle ayant de 3 à 8 atomes de carbone, éventuellement substitué, aryle, éventuellement substitué,
R₂ est hydrogène, alcoyle ayant de 1 à 6 atomes de carbone et
R₃ est phényle, éventuellement substitué par un substituant choisi parmi alcoyle ayant de 1 à 6 atomes de carbone, alcoxy ayant de 1 à 6 atomes de carbone, halogène, hydroxyle, NO₂
pour la fabrication d'un médicament pour le traitement d'une maladie hyperproliférative ou d'une transformation maligne impliquant une protéine kinase.

14. Utilisation suivant la revendication 13, dans laquelle
R₁ est cyclopentyle, cyclohexyle, phényle, éventuellement substitué par un substituant choisi parmi méthyle, éthyle, propyle ; (CH₂)ₙCO(O)R₄ dans laquelle n = 1 et dans laquelle R₄ est méthyle, éthyle, propyle,
R₂ est hydrogène et
R₃ est phényle, éventuellement substitué par un substituant choisi parmi méthoxy, éthoxy, hydroxy, NO₂, brome, fluor, chlore.

15. Utilisation selon la revendication 13 ou 14, dans laquelle
R₁ est phényle, R₂ est hydrogène et R₃ est phényle substitué par hydroxyle et
R₁ est phényle, R₂ est hydrogène et R₃ est phényle.

16. Composé de formule Ib en tant que médicament 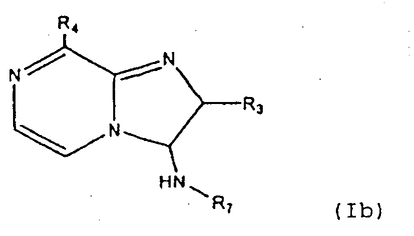 R₇ est phényle, éventuellement substitué,
R₃ est phényle, éventuellement substitué ou furyle éventuellement substitué de préférence non substitué, et
R₄ est méthyle ou NR₆R₇ dans laquelle R₆ et R₇ sont choisis indépendamment l'un de l'autre parmi l'hydrogène, alcoyle ayant 1 ou 2 atome de carbone, éventuellement substitué par phényle.

17. Composé selon la revendication 16, dans lequel
R₇ est phényle non substitué ou phényle substitué en la position 3 et/ou en la position 4 par un ou par deux substituants choisis indépendamment parmi halogène, alcoxy ayant 1 ou 2 atome de carbone, en particulier méthoxy, alcoyle ayant 1 ou 2 atome de carbone, en particulier méthyle, CF₃, NO₂, NH₂, NHCH₃, N(CH₃)₂,
R₃ est phényle non substitué ou phényle substitué en la position 3 et/ou en la position 4 par un substituant choisi indépendamment parmi alcoxy ayant de 1 à 4 atomes de carbone, ou hydroxy, ou amino, ou les substituants en la position 3 et 4 forment ensemble un hétérocyle à 5 ou 6 chaînons, et
R₄ est méthyle.

18. Composé suivant la revendication 17, dans lequel
R₇ est phényle non substitué ou phényle substitué en la position 3 et/ou en la position 4 par un ou par deux substituants choisi indépendamment parmi halogène, et CF₃, en particulier phényle non substitué ou phényle substitué en la position 3 ou en la position 4,
R₃ est phényle non substitué ou phényle substitué en la position 3 et/ou en la position 4 par méthoxy ou hydroxy, en particulier phényle non substitué ou phényle substitué en la position 3 ou en la position 4,
R₄ est méthyle.

19. Composé suivant la revendication 18, dans lequel
R₇ est phényle substitué par halogène en la position 3,
R₃ est phényle non substitué, et
R₄ est méthyle.

20. Composé de formule 3, 24, 33
